# EUROPÄISCHE PATENTSCHRIFT

(11) **EP 4 237 831 B1**
(45) Veröffentlichungstag und Bekanntmachung des Hinweises auf die Patenterteilung: **03.07.2024**
(21) Anmeldenummer: 21802671.4
(22) Anmeldetag: 29.10.2021
(51) Int. Cl.: G01N 21/53, G01N 21/49, G01N 15/06, G01N 21/85, G01N 33/28, G01N 15/00

(54) **ÖLNEBELDETEKTOR ZUR DETEKTION UND/ODER ANALYSE VON ÖL-LUFTGEMISCHEN MIT EINER OPTISCHEN MESSANORDNUNG SOWIE ZUGEHÖRIGE VERFAHREN**
OIL-MIST DETECTOR HAVING AN OPTICAL SENSOR FOR DETECTION AND/OR ANALYSIS OF OIL-AIR MIXTURES AND CORRESPONDING METHOD
DÉTECTEUR DE BROUILLARD D`HUILE AYANT UNE SONDE OPTIQUE POUR DÉTECTION ET/OU ANALYSE DE MÉLANGES AIR-HUILE ET PROCÉDÉ CORRESPONDANT

(30) Priorität: 30.10.2020 DE 102020128684
(43) Veröffentlichungstag der Anmeldung: 06.09.2023
(73) Patentinhaber: Heinzmann GmbH & Co. KG, 79677 Schönau (DE)
(72) Erfinder: HANS, Hermann, 79677 Schönau (DE); LEVCHENKO, Oleksandr, 79677 Schönau (DE); WOLF, Jens Wilhelm, 79677 Schönau (DE)
(74) Vertreter: Mertzlufft-Paufler, Cornelius
(86) Internationale Anmeldenummer: PCT/EP2021/080205
(87) Internationale Veröffentlichungsnummer: WO 2022/090511

(56) Entgegenhaltungen:
- EP-A1- 3 086 109
- EP-A2- 0 071 391
- EP-B1- 1 729 108
- WO-A2-2009/095679
- DE-U1-202010 010 042
- US-A- 4 672 216
- US-A- 5 352 901
- US-B2- 10 359 365

## Beschreibung

Die Erfindung betrifft einen Ölnebeldetektor zur Detektion und/oder Analyse von Öl-Luft-Gemischen. Solche Gemische können als potentiell explosives Gemisch oder als ein (auch subexplosives) Gemisch mit einem charakterisierten Mischungsverhältnis vorliegen. Diese Gemische, die häufig in Form von Dispersionen von feinen Öltröpfchen in Luft vorkommen, werden auch als Ölnebel oder Aerosol bezeichnet. Zur Detektion und/oder Analyse des Öl-Luft-Gemischs, insbesondere von potentiell explosivem feinem Ölnebel, weist der Ölnebeldetektor eine Messkammer auf, die mindestens eine optische Messstrecke zur optischen Detektion eines Ölnebels ausbildet. Mithilfe der Messkammer kann der Ölnebeldetektor somit potentiell explosionsfähige Gemische oder Ölnebel erkennen, ein entsprechendes Warnsignal ausgeben und somit für eine erhöhte Betriebssicherheit sorgen.

Der Ölnebeldetektor kann dabei insbesondere so ausgestaltet sein, dass er in ein Kurbelraumgehäuse einer Kolbenmaschine (wie etwa ein Verbrennungsmotor, oder ein Kompressor) einsetzbar ist, um in dem Kurbelraumgehäuse entstehende feine Öltröpfchen frühzeitig detektieren zu können, da diese mit Luft ein explosives Gemisch in dem Kurbelraum bilden können.

Die Erfindung betrifft ferner eine Kolbenmaschine, die insbesondere als Kompressor oder Verbrennungsmotor ausgestaltet sein kann, mit einem Kurbelraumgehäuse, welches einen Kurbelraum begrenzt, und mit einem wie eingangs beschriebenen Ölnebeldetektor.

Schließlich betrifft die Erfindung ein Verfahren zum Betrieb eines Ölnebeldetektors wie zuvor beschrieben, insbesondere ein Verfahren zur Detektion und/oder Analyse von Öl-Luftgemischen in einem überwachten Raum wie beispielsweise einem Innenraum, insbesondere Kurbelraum, einer Kolbenmaschine (z.B. eines Verbrennungsmotors) mit Hilfe eines Ölnebeldetektors, der eine Messkammer aufweist, die eine optische Messstrecke zur optischen Detektion und/oder Analyse eines Ölnebels ausbildet.

Die Detektion und Analyse von Öltröpfchen in Luft mit Hilfe von Licht sind grundsätzlich bekannt. Insbesondere sind Ölnebeldetektoren bekannt, die mittels Durchlicht- und/oder Streulicht-Messungen wie eingangs beschriebene Ölnebel detektieren können. So sind etwa für die Erfindung relevante optische Vorrichtungen aus DE 20 2010 010 042 U1, US 5 352 901 A, WO 2009 095679 A2, US 10 359 365 B2, EP 3 086 109 A1, EP 0 071 391 A2, US 4 672 216 A und aus EP 1 729 108 B1 vorbekannt.

Die Entstehung beziehungsweise Detektion von kleinen Öltröpfchen innerhalb eines überwachten Raums wie etwa eines Motorraums ist insofern relevant, als das Vorhandensein derartiger Öltröpfchen darauf rückschließen lässt, dass in naher Zukunft ein Ausfall des Motors zu erwarten ist. Im schlimmsten Fall kann es nämlich dazu kommen, dass die Öltröpfchen sich an durch Reibungswärme erhitzten Oberflächen oder sonstigen Zündquellen entzünden und so eine Explosion in dem überwachten Raum auslösen. Ähnliche Gefahren treten beispielsweise auch in Anlagen der Prozessindustrie auf und auch hier kann eine erfindungsgemäßer Ölnebeldetektor sinnvoll zur Überwachung eines Raums eingesetzt werden.

Es kann hierbei unterschieden werden zwischen mechanisch erzeugtem Ölnebel, der aus relativ großen Öltröpfchen mit Durchmessern von 50 µm oder mehr besteht und feinem Ölnebel, der aufgrund von heißen Oberflächen oder auf andere Weise durch Verdampfen erzeugt wird, wobei diese Art von Nebel typischerweise eine Tröpfchengröße von kleiner 10 µm, beispielsweise von 1-5 µm, aufweist. Diese kleinen Tröpfchen sind sehr gefährlich, da sie besonders leicht in Luft zur Bildung von potentiell explosiven Gemischen führen können.

In typischen Anwendungssituationen entstehen jedoch sowohl diese kleinen Tröpfchen als auch die großen Öltröpfchen und bilden gemeinsam ein inhomogenes Öl-Luft-Gemisch, das optisch untersucht werden muss.

Die Erfindung hat es sich vor diesem Hintergrund zur Aufgabe gemacht, die Detektion und/oder Analyse der kleineren Öltröpfchen in solchen Öl-Luft-Gemischen zu verbessern, insbesondere zur sicheren Überwachung des Betriebs von Kolbenmaschinen, insbesondere von Verbrennungsmotoren. Dadurch soll die Zuverlässigkeit in der Detektion potentiell explosiver Öl-Luft-Gemische erhöht werden. Insbesondere soll ein sicherer Betrieb des Ölnebeldetektors über Jahre ermöglicht werden.

Zur Lösung dieser Aufgabe sind erfindungsgemäß bei einem Ölnebeldetektor die Merkmale von Anspruch 1 vorgesehen. Insbesondere wird somit erfindungsgemäß zur Lösung der Aufgabe bei einem Ölnebeldetektor der eingangs genannten Art vorgeschlagen, dass die Messkammer eine optische Messanordnung von Lichtquellen und Lichtdetektoren aufweist und dass diese optische Messanordnung zur Durchführung einer gleichzeitigen Durchlichtmessung und einer Streulichtmessung, jeweils an einer in der Messkammer befindlichen Gasprobe, eingerichtet ist.

Vorteilhaft an einer solchen Ausgestaltung ist zunächst, dass Fehlalarme erkannt und entsprechend unterdrückt werden können, die bei Eindringen von Wassertröpfchen in die Messkammer entstehen. Hingegen kann ein Vorhandensein einer Dispersion aus kleinen Öltröpfchen in Luft in der Messkammer sicher erkannt werden und zwar selbst dann, wenn auch Wassernebel in der Messkammer vorhanden ist. Denn durch die Verwendung einer zusätzlichen Streulichtmessung stehen zusätzliche Messparameter zur Verfügung, die eine Differenzierung zwischen Signalen, die von Öltröpfchen herrühren und solchen Signalen, die von dem Wassernebel verursacht werden, erlauben. Die Ursache hierfür ist, dass Wassernebel und Ölnebel Licht unterschiedlich stark streuen.

Wie eingangs erläutert wurde, sind gerade solche Dispersionen von feinen Öltröpfchen mit Durchmessern von weniger als 10 µm in Luft für eine eventuelle Explosionsgefahr verantwortlich, sodass gerade diese Art von feinem Ölnebel sicher detektiert werden muss, um die Sicherheit im Betrieb zu gewährleisten.

Für eine schnelle optische Charakterisierung und entsprechend schnelle Detektion von gerade entstehenden, insbesondere potentiell explosiven, Ölnebeln in einem von dem Ölnebeldetektor überwachten Raum ist es gemäß der Erfindung vorteilhaft, wenn die Streulichtmessung und die Durchlichtmessung gleichzeitig, insbesondere unter Verwendung derselben Lichtquelle/Lichtquellen, durchgeführt wird. Mit anderen Worten kann somit sowohl die Durchlichtmessung als auch die Streulichtmessung auf Licht basieren, welches von einer Lichtquelle der Messanordnung zu einem bestimmten Zeitpunkt ausgesendet worden ist.

Wie noch genauer zu erläutern sein wird, können zudem sowohl die Durchlicht- als auch die Streulichtmessung jeweils als Redundantmessung unter Verwendung jeweils mindestens zweier Transmissionslichtpfade beziehungsweise mindestens zweier Streulichtpfade, ausgestaltet sein. Unter einer Redundantmessung kann hier insbesondere eine optische Messung verstanden werden, bei welcher ein identischer Anteil eines Gesamtvolumens einer in der Messkammer befindlichen Gasprobe mittels Licht durch mindestens zwei unabhängige optische Messungen untersucht wird.

Weist die optische Messanordnung beispielsweise zwei Streulichtdetektoren auf, kann eine zusätzliche Redundanz erhalten werden, sodass insbesondere Fehlalarme vermieden werden können, indem der Ölnebeldetektor eine Plausibilitätsprüfung von Messergebnissen durchführt, die in mit den beiden Streulichtdetektoren durchgeführten Streulichtmessungen gewonnen wurden.

Zur Unterdrückung von unerwünschten Lichtreflexionen und daraus resultierenden falschen Messsignalen kann die Messkammer bevorzugt schwarz eloxiert sein. Ferner kann die Messkammer innenseitig, etwa mittels Beschichtungen oder geeignete Materialwahl, so ausgestaltet werden, dass diese Infrarotstrahlung absorbiert, um so die Qualität der Messsignale zu verbessern.

Erfindungsgemäß kann die Aufgabe auch durch weitere vorteilhafte Ausführungen der Messanordnung gelöst werden, die im Folgenden beschrieben werden:
So umfasst die Messanordnung erfindungsgemäß ein erstes Funktionspaar und ein zweites Funktionspaar aus je einer Lichtquelle und einem Lichtdetektor. Hierbei kann die jeweilige Lichtquelle und der jeweilige Lichtdetektor eines Funktionspaars durch ein und dasselbe optische Bauteil gebildet sein (etwa eine LED, die wechselweise als Lichtquelle und als Lichtdetektor betrieben wird). Oder aber, die Lichtquelle und der Lichtdetektor eines Funktionspaars kann jeweils als ein separates optisches Bauteil gebildet sein. Diese Alternativen können auch miteinander kombiniert werden.

Die Ausgestaltung von separaten optischen Bauteilen für die jeweilige Lichtquelle und den jeweiligen Lichtdetektor hat dabei den Vorteil, dass das betroffene Funktionspaar nicht nur wechselweise, sondern auch gleichzeitig Licht aussenden und empfangen kann.

Ferner können bei separater Ausgestaltung von Lichtquelle und Lichtdetektor die jeweilige Lichtquelle und der jeweilige Lichtdetektor eines Funktionspaars zu einer kompakten Baueinheit zusammengefasst sein. Dadurch lässt sich das Funktionspaar besonders einfach austauschen und die Justage beim Auswechseln kann wesentlich erleichtert werden.

Hierbei kann jeweils die Lichtquelle des jeweiligen Funktionspaars in Verbindung mit dem Lichtdetektor des jeweils anderen Funktionspaars eine optische Messtrecke für eine Durchlichtmessung ausbilden. Das heißt die beiden Baueinheiten/Funktionspaare können ein bidirektionales Messpaar bilden, welches zwei redundante, insbesondere gleichzeitige, Durchlichtmessungen eines identischen Gasvolumens in der Messkammer ermöglicht.

So ist erfindungsgemäß vorgesehen, dass die Lichtquelle des ersten Funktionspaars mit dem Lichtdetektor des zweiten Funktionspaars eine erste optische Messtrecke ausbildet, während die Lichtquelle des zweiten Funktionspaars mit dem Lichtdetektor des ersten Funktionspaars eine zweite optische Messtrecke ausbildet. Hierbei verlaufen die erste und die zweite optische Messstrecke in zueinander entgegengesetzten Richtungen, das heißt insbesondere parallel zueinander. Durch eine solche Ausgestaltung ist es möglich, dass die erste und die zweite optische Messstrecke gleichzeitig auswertbar sind, was die Messsicherheit wesentlich erhöht.

Dies ist beispielsweise besonders einfach möglich, wenn das erste und das zweite Funktionspaar einander gegenüberliegend angeordnet sind und/oder die erste und zweite optische Messstrecke durch die Messkammer hindurch verlaufen. Bei einer solchen Messanordnung kann also die Lichtquelle des ersten Funktionspaars auf den Lichtdetektor des zweiten Funktionspaars ausgerichtet sein, während der Lichtdetektor des ersten Funktionspaars auf die Lichtquelle des zweiten Funktionspaars ausgerichtet sein kann.

Für einen einfachen Aufbau der Messanordnung sowie eine einfache Justage ist es ferner vorteilhaft, wenn die beiden Funktionspaare jeweils baulich als eine Einheit ausgestaltet sind, die jeweils in eine Aufnahme der Messkammer, vorzugsweise herausnehmbar, eingesetzt ist. Hierbei ist es vorzuziehen, wenn sich die beiden Aufnahmen derart gegenüberliegen und/oder zueinander ausgerichtet sind, dass die erste und zweite optische Messstrecke durch die Messkammer verlaufen, vorzugsweise durch ein Zentrum der Messkammer.

Diese beiden Funktionspaare können somit zwei redundante Durchlichtmessungen, nämlich mit Hilfe der ersten und der zweiten optischen Messstrecke, realisieren. Dies gelingt besonders einfach, wenn die beiden Lichtquellen und die beiden Lichtdetektoren jeweils achsensymmetrisch zu einer gemeinsamen Symmetrieachse angeordnet sind. Diese Symmetrieachse kann bevorzugt durch das Zentrum der Messkammer verlaufen.

Zur Streulichtmessung kann die optische Messanordnung nun ferner mindestens einen weiteren Lichtdetektor, bevorzugt jedoch mindestens zwei weitere Lichtdetektoren, aufweisen. Diese können als Streulichtdetektoren fungieren. Der weitere Lichtdetektor kann beziehungsweise diese weiteren Lichtdetektoren können jeweils eine Sichtlinie ausbilden, welche einen Winkel von mindestens 10°, bevorzugt von mindestens 30°, zur ersten und/oder zweiten optischen Messstrecke einschließt. Unter einer Sichtlinie kann hierbei eine Richtung verstanden werden, aus welcher der jeweilige Lichtdetektor Licht empfangen kann. Diese Sichtlinie kann also gerade demjenigen Lichtpfad entsprechen, welche Photonen zurücklegen, die an Tröpfchen in der Messkammer gestreut werden und zum jeweiligen weiteren Lichtdetektor gelangen.

Um die Sicherheit der Streulichtmessung zu verbessern und damit das Auftreten von Fehlalarmen zu vermeiden, kann insbesondere mindestens ein zweiter weiterer Lichtdetektor zur Durchführung mindestens einer zweiten Streulichtmessung vorgesehen sein. Diese mindestens zweite Streulichtmessung(en) kann/können dabei insbesondere redundant sein zu einer mittels des (ersten) weiteren Lichtdetektors durchgeführten ersten Streulichtmessung.

Vorzugsweise können demnach zwei separate Streulichtdetektoren vorgesehen sein sowie zwei Funktionspaare aus je einer Lichtquelle und einem Durchlichtdetektor. Hierbei ist es bevorzugt, wenn die beiden Funktionspaare diametral angeordnet sind.

Der Ölnebeldetektor kann ferner eine Berechnungseinheit aufweisen, die dazu eingerichtet ist, zwei unabhängige Durchlichtmessungen mittels der ersten und zweiten optischen Messstrecke, insbesondere gleichzeitig, auszuwerten. Dadurch können also redundante Messergebnisse in Bezug auf eine Durchlichtmessung einer in der Messkammer befindlichen Gasprobe erzeugt und damit eine hohe Sicherheit der Durchlichtmessung erzeugt werden.

Der Ölnebeldetektor kann ferner eine, insbesondere die zuvor erläuterte, Berechnungseinheit aufweisen, insbesondere in Form einer Elektronik-Einheit, die dazu eingerichtet ist, eine Lichtabsorption auf Basis der Durchlichtmessung sowie eine Streulichtintensität auf Basis der Streulichtmessung quantitativ zu bestimmen. Denn eine gemessene Transmission/Lichtabsorption in Verbindung mit einer Streulichtintensität, die jeweils, vorzugsweise gleichzeitig, an einer Gasprobe in der Messkammer bestimmt wurde, kann zur sicheren Charakterisierung der Gasprobe eingesetzt werden und insbesondere zur Beantwortung der Frage, ob es sich bei der Gasprobe um Wassernebel und/oder um Ölnebel handelt.

Genauer kann die Berechnungseinheit dazu eingerichtet sein, anhand der bestimmten Lichtabsorption und der bestimmten Streulichtintensität Messsignale, die einem Wasseranteil einer Gasprobe in der Messkammer zuordenbar sind, von Messsignalen zu unterscheiden, die einem Anteil eines Aerosols oder Dispersion von Öltröpfchen, insbesondere von weniger als 10 µm Durchmesser, der Gasprobe zuordenbar sind.

Hierbei kann die Berechnungseinheit zudem dazu eingerichtet sein, die bestimmte Streulichtintensität zur Differenzierung der beiden Messsignale zu verwenden und/oder dazu, auf Basis der bestimmten Lichtabsorption und/oder der bestimmten Streulichtintensität auf die Anwesenheit des Aerosols oder der Dispersion von (kleinen) Öltröpfchen zu schließen und ein entsprechendes Warnsignal auszugeben.

Mit anderen Worten kann der Ölnebeldetektor somit dazu eingerichtet sein, anhand von Messsignalen der Streulichtmessung und der Durchlichtmessung ein in der Kammer befindliche Gasprobe daraufhin zu charakterisieren, ob die Gasprobe einen, insbesondere für die Betriebssicherheit kritischen, Ölnebel enthält oder nicht, und zwar insbesondere in Situationen, in denen die Gasprobe auch einen Wasseranteil enthält. Der Ölnebeldetektor kann somit dazu eingerichtet sein, anhand von Messsignalen der Streulichtmessung und der Durchlichtmessung Messsignalanteile, die von einem Wasseranteil einer in der Messkammer befindlichen Gasprobe herrühren von Messsignalanteilen zu differenzieren, die von einem Öl-Anteil der Gasprobe verursacht sind.

Zu diesem Zweck kann die Berechnungseinheit insbesondere dazu eingerichtet sein, anhand von mit der zweiten optischen Messstrecke gewonnenen Messdaten eine Plausibilitätsprüfung durchzuführen in Bezug auf Messdaten, die mit der ersten optischen Messstrecke gewonnen wurden. Dadurch kann also die Sicherheit der Messdaten aus der Durchlichtmessung erhöht werden.

Alternativ oder ergänzend kann die Berechnungseinheit auch dazu eingerichtet sein, anhand von mit der zweiten Streulichtmessung gewonnenen Messdaten eine Plausibilitätsprüfung durchzuführen in Bezug auf Messdaten, die mit der ersten Streulichtmessung gewonnen wurden. Dadurch kann also die Sicherheit der Messdaten aus der Streulichtmessung erhöht werden.

Die folgenden Merkmale des Ölnebeldetektors können unterstützend zu den zuvor in Bezug auf die Ausgestaltung der optischen Messkammer erläuterten Merkmalen der Erfindung eingesetzt werden.

Eine mögliche Ausgestaltung sieht bei dem eingangs erwähnten Ölnebeldetektor vor, dass die Messkammer mittels einer Gaszuführung mit einem Ansaugkanal verbunden ist, der in einer Ansaugöffnung mündet, dass ferner eine Gasabführung vorgesehen ist zum Abführen von Gas aus der Messkammer und dass schließlich ein Bypass vorgesehen ist zum Abführen von Gas aus dem Ansaugkanal unter Umgehung der Messkammer. Hierbei ist es für eine kompakte Bauweise vorzuziehen, wenn der Bypass die Gaszuführung mit der Gasabführung verbindet. Zudem sei an dieser Stelle bereits erwähnt, dass die Ansaugöffnung in einer Einbausituation des Ölnebeldetektors bevorzugt innerhalb eines mit dem Ölnebeldetektors überwachten Raums, beispielsweise eines untersuchten Innenraums, angeordnet sein kann.

Das Vorsehen eines derart ausgestalteten Bypasses ermöglicht eine effiziente Separation von feinsten Öltröpfchen aus einer mit dem Ölnebeldetektor aufgenommenen Gasprobe in die Messkammer, die dann dort optisch präzise und sicher vermessen werden können, um so insgesamt eine hohe Sicherheit in der Bestimmung dieser kleinen Öltröpfchen zu erreichen. Insofern besteht eine Synergie darin, dass die Separation der feinen Öltröpfchen überhaupt erst eine Differenzierung zwischen feinem Ölnebel und Wassernebel in der Messkammer ermöglicht.

Die Umgehung der Messkammer mittels des Bypasses hat dabei insbesondere den Vorteil, dass schwerere Öltröpfchen, insbesondere solche von mehr als 10 µm Durchmesser, durch den Bypass abgeführt werden können, um so zu verhindern, dass diese schwereren Öltröpfchen in die Messkammer gelangen und dort die optische Messung von feineren Öltröpfchen behindern oder gar verunmöglichen. Der Bypass scheidet somit größere Ölpartikel, insbesondere größere flüssige Öltropfen, ab, sodass diese nicht in die Messkammer gelangen. Dadurch wird insbesondere verhindert, dass sich solche größeren Ölpartikel auf den Lichtquellen und Lichtdetektoren in der Messkammer absetzen und diese verschmutzen, was die eigentlich optische Messung beeinträchtigen würde.

Der Bypass kann somit einen ersten Teilgasstrom aus dem Ansaugkanal abzweigen und aus diesem abführen. Die Gaszuführung kann hingegen einen zweiten, insbesondere restlichen Teilgasstrom, aus dem Ansaugkanal abzweigen und in die Messkammer führen. Die Gasabführung wiederum kann den zweiten, insbesondere restlichen, Teilgasstrom, nachdem dieser die Messkammer durchströmt hat, aus der Messkammer abführen. Hierbei kann der erste Teilgasstrom überwiegend schwerere Öltröpfchen aufweisen, während der zweite Teilgasstrom überwiegend kleinere Öltröpfchen aufweisen kann.

Sofern der Bypass die Gaszuführung mit der Gasabführung verbindet, kann der Bypass den ersten Teilgasstrom und den zweiten, insbesondere restlichen, Teilgasstrom, in der Gasabführung vereinigen.

Alternativ oder ergänzend kann auch vorgesehen sein, dass der Bypass und die Gasabführung aus der Messkammer in eine Gasfördervorrichtung des Ölnebeldetektors münden. Somit kann die Gasfördervorrichtung sowohl Gas durch den Bypass als auch durch die Messkammer und die sich an die Messkammer in Gasströmungsrichtung anschließende Gasabführung fördern. Somit kann also mit dem Ölnebeldetektor ein durch die Ansaugöffnung einströmender Gasstrom in mindestens zwei Teilgasströme aufgeteilt werden, wobei lediglich einer der beiden Teilgasströme durch die Messkammer gefördert wird.

Durch den Bypass kann somit ein Teil eines durch die Ansaugöffnung einströmenden Gasstroms als ein erster Teilgasstrom an der Messkammer vorbei geführt werden. Dadurch kann die optische Messung in der Messkammer unbeeinflusst von diesem durch den Bypass strömenden ersten Teilgasstrom ausgeführt werden, was vorteilhaft für eine hohe Messsicherheit ist.

Mit anderen Worten schlägt die Erfindung somit vor,
- dass der Ölnebeldetektor einen Ansaugkanal aufweist zum Ansaugen einer Gasprobe, beispielsweise direkt aus einem Kurbelraumgehäuse eines Verbrennungsmotors, durch eine Ansaugöffnung;
- eine Gaszuführung zum Führen zumindest eines Teils der Gasprobe von dem Ansaugkanal in die Messkammer;
- einen Bypass, beispielsweise in Form eines Verbindungskanals ausgebildet, durch welchen ein erster Teil der Gasprobe direkt, also insbesondere unter Umgehung der Messkammer, von dem Ansaugkanal abgeführt werden kann;
- und eine Gasabführung, mit der ein zweiter, insbesondere restlicher, Teil der Gasprobe, vorzugsweise in einem Durchströmungs-Betrieb, aus der Messkammer abführbar ist;

Wie zuvor bereits erläutert, kann hierbei der Bypass in diese Gasabführung münden.

Ein solcher Ölnebeldetektor kann demnach insbesondere mit Hilfe der zuvor beschriebenen Ausgestaltungen dazu eingerichtet sein, eine einströmende Gasprobe, also ein bestimmtes Volumen an Gas beziehungsweise eines Gasgemischs, welches optisch vermessen werden soll, aufzuteilen in
- einen ersten Teilgasstrom, der einen Bypass, unter Umgehung der Messkammer, passiert, und
- einen zweiten, insbesondere restlichen, Teilgasstrom, der eine Gaszuführung und anschließend die besagte optische Messkammer passiert. Der Ölnebeldetektor kann ferner dazu eingerichtet sein, die beiden Teilgasströme anschließend in einer gemeinsamen Gasabführung zusammenzuführen.

Durch die Aufteilung der Gasprobe in zwei getrennte Teilgasströme lässt sich die eigentliche optische Messung wesentlich verbessern. Denn mittels des Bypass kann im Messbetrieb ein Teil eines Gasstroms, der durch den Ansaugkanal strömt, als ein erster Teilgasstrom an der Messkammer vorbei in die Gasabführung geleitet werden. Dadurch kann vermieden werden, dass dieser erste Teilgasstrom des durch die Ansaugöffnung einströmenden Gasstroms in die Messkammer gelangt.

Die Ansaugöffnung kann in der Einbausituation insbesondere innerhalb eines Kurbelraumgehäuses, also insbesondere in einem Kurbelraum, eines Verbrennungsmotors angeordnet sein, der mit Hilfe des Ölnebeldetektors überwacht wird.

Durch die zuvor beschriebene Gasstromführung kann erreicht werden, dass größere Öltröpfchen, insbesondere solche von mindestens 10 µm Durchmesser, die im durch die Ansaugöffnung einströmenden Gasstrom enthalten sind, bevorzugt durch den Bypass strömen und nur vereinzelt oder im besten Fall überhaupt nicht in die Messkammer gelangen. Ferner kann erreicht werden, dass kleinere Öltröpfchen, insbesondere solche von weniger als 10 µm Durchmesser oder gar von weniger als 5 µm Durchmesser - sofern diese ebenfalls im durch die Ansaugöffnung einströmenden Gasstrom enthalten sind - bevorzugt durch die Gaszuführung in die Messkammer gelangen, um dort optisch vermessen werden zu können.

Mit anderen Worten ermöglicht das Vorsehen eines erfindungsgemäß ausgestalteten Bypass somit eine Separation von feinem Ölnebel, der in dem durch die Ansaugöffnung einströmenden Gasstrom enthalten ist, von gröberen Öltröpfchen. Diese Separation erhöht die Sicherheit in der optischen Detektion von potentiell explosiven Gemischen erheblich, da die größeren Öltröpfchen die genaue Detektion von kleineren Öltröpfchen stören können.

Erfindungsgemäß kann die Aufgabe auch noch durch weitere vorteilhafte Ausführungen, insbesondere in Kombination mit zuvor erläuterten Merkmalen in Zusammenhang mit der erfindungsgemäßen Messanordnung und/oder dem erfindungsgemäßen Bypass, gelöst werden. Diese werden im Folgenden beschrieben:
Beispielsweise schlägt eine Ausgestaltung vor, dass der Bypass an einer Verzweigungsstelle von dem Ansaugkanal so abgezweigt ist, dass ein erster Teilgasstrom eines durch die Ansaugöffnung und den Ansaugkanal strömenden Gasstroms, durch den Bypass hindurch und an der Messkammer vorbei in eine Gasabführung, insbesondere die zuvor erläuterte Gasabführung aus der Messkammer, leitbar ist.

Bei einer solchen Ausgestaltung kann ferner ein zweiter Teilgasstrom des Gasstroms, der insbesondere ein restlicher Teil des Gasstroms sein kann, durch die Gaszuführung in die Messkammer leitbar sein.

Bei einer solchen Ausgestaltung kann somit der durch die Ansaugöffnung einströmende Gasstrom in einen ersten Teilgasstrom, der durch den Bypass unter Umgehung der Messkammer strömt, und einen restlichen Teilgasstrom, der in die Messkammer gelangt, aufgeteilt werden.

Hierbei ist es bevorzugt, wenn die Gaszuführung an der Verzweigungsstelle von dem Ansaugkanal, bezogen auf eine Einbausituation des Ölnebeldetektors, nach oben abzweigt. Denn in diesem Fall können die kleineren Öltröpfchen nach oben, also entgegen der Schwerkraft, durch die Gaszuführung aus dem durch den Ansaugkanal einströmenden Gasstrom abgesaugt und somit von größeren im Gasstrom enthaltenen Öltröpfchen separiert werden. Die Abzweigung nach oben hat dabei den Vorteil, dass die schwereren Öltröpfchen tendenziell aufgrund ihrer größeren Trägheit nach unten wandern und daher weniger häufig oder überhaupt nicht in die Gaszuführung und damit in die Messkammer gelangen.

In einer Einbausituation, also beispielsweise dann, wenn der Ölnebeldetektor in ein Kurbelraumgehäuse eines Verbrennungsmotors eingesetzt ist, kann die Gaszuführung somit den zweiten, insbesondere restlichen, Teilgasstrom entgegen der Schwerkraft und/oder entgegen einer Strömungsrichtung des ersten Teilgasstroms von dem Ansaugkanal abführen. Es versteht sich, dass bei einer solchen Ausgestaltung der erste Teilgasstrom überwiegend größere Öltröpfchen, insbesondere von mindestens 10 µm Durchmesser, aufweisen kann. Denn diese Tröpfchen haben ein vergleichsweise kleines Verhältnis von Oberfläche zu Gewicht und werden daher leichter in Richtung der Schwerkraft absinken beziehungsweise leichter in Richtung der Strömungsrichtung des ersten Teilgasstroms weiterfliegen als Tröpfchen von kleinerem Durchmesser.

Ferner kann die Abführung des restlichen Teils des Gasstroms dadurch so ausgestaltet sein, dass der zweite, insbesondere restliche, Teilgasstrom, welcher in die Messkammer gelangt, arm ist an größeren Öltröpfchen, vorzugsweise von mehr als 10 um Durchmesser, verglichen mit dem Anteil dieser größeren Öltröpfchen in dem durch den Ansaugkanal einströmenden Gasstrom. In einem solchen Fall kann der zweite Teilgasstrom somit beispielsweise überwiegend kleinere Öltröpfchen, vorzugsweise von weniger als 10 µm Durchmesser, aufweisen.

Für eine besonders effiziente Separation von Öltröpfchen unterschiedlicher Größe ist es vorteilhaft, wenn vor der Verzweigungsstelle in dem Ansaugkanal ein Beschleunigungsabschnitt zum Beschleunigen eines durch die Ansaugöffnung einströmenden Gasstroms entlang einer Beschleunigungsrichtung ausgebildet ist. Dieser Beschleunigungsabschnitt kann genutzt werden, um schwerere Öltröpfchen, insbesondere solche von mehr als 10 µm Durchmesser, in den Bypass abzusondern und so nicht in die Messkammer gelangen zu lassen. Diese konstruktive Ausgestaltung führt im Ergebnis zur Ausbildung eines Trägheitsabscheiders, der schwerere Öltröpfchen oder sonstigen Schmutz in den Bypass absondert, sodass diese nicht in die Messkammer gelangen.

Die erfindungsgemäße Separation erhöht somit den relativen Anteil von kleineren Öltröpfchen in der Messkammer bezogen auf den relativen Anteil dieser kleineren Öltröpfchen in dem durch die Ansaugöffnung einströmenden Gasstrom, welcher in typischen Anwendungssituationen auch einen nennenswerten Anteil an größeren Öltröpfchen aufweisen kann. Somit wird der Anteil dieser größeren Öltröpfchen in der Messkammer vermindert, was vorteilhaft ist für eine zuverlässige Messung.

Mit anderen Worten wird durch die erfindungsgemäße Separation erreicht, dass - nach der erfolgten Absonderung der kleineren Öltröpfchen in die Gaszuleitung - der erste Teilgasstrom, welcher durch den Bypass strömt, einen erhöhten relativen Anteil an größeren Öltröpfchen aufweist. Demgegenüber weist dann der zweite Teilgasstrom, welcher in die Messkammer strömt, einen erhöhten relativen Anteil an kleineren Öltröpfchen, insbesondere mit Durchmessern von 10 µm und weniger, auf. (Dies jeweils im Vergleich zu dem einströmenden Gasstrom, in welchem sowohl kleinere als auch größere Öltröpfchen enthalten sein können). Mit anderen Worten werden somit die größeren Öltröpfchen in den Bypass abgeschieden. Hierbei steigt die Effizienz dieser Abscheidung / Separation mit der Größe dieser Öltröpfchen stark an.

Der Beschleunigungsabschnitt kann beispielsweise durch eine Verengung eines Strömungsquerschnitts gebildet sein. Dies kann in einer Zunahme der Strömungsgeschwindigkeit des Gasstroms resultieren und damit in einer Zunahme der kinetischen Energie der einzelnen Öltröpfchen. Der besagte Beschleunigungsabschnitt kann somit insbesondere den zuvor erläuterten ersten Teilgasstrom beschleunigen, welcher durch den Bypass geleitet wird. Gleichzeitig kann der Beschleunigungsabschnitt auch den zweiten Teilgasstrom beschleunigen, welcher in die Messkammer eintritt. Durch die Beschleunigung wird dabei die zuvor beschriebene Separation der größeren Öltröpfchen verbessert.

Hierbei ist es für eine effiziente Separation mit Hilfe der Schwerkraft in einer Einbausituation des Ölnebeldetektors von Vorteil, wenn der Beschleunigungsabschnitt in Richtung des besagten Gasstroms einen Anstieg ausbildet. Hierzu kann der Beschleunigungsabschnitt beispielsweise einen Kanalboden aufweisen, welcher in der Einbausituation in Richtung der Beschleunigungsrichtung ansteigt.

Der Ölnebeldetektor kann ferner so ausgestaltet sein, dass die Messkammer in einer Einbausituation am höchsten liegt (im Vergleich zu Gaszuführung und Gasabführung), während die Gaszuführung und die Gasabführung von unten in die Messkammer münden. Bei einer solchen Ausgestaltung steigt der in die Messkammer geführte Teilgasstrom somit zunächst durch die Gaszuführung auf, um nach Passieren der Messkammer durch die Gasabführung wieder abzusteigen.

Vorteilhaft an dieser Ausgestaltung ist, dass - insbesondere bei Abnahme oder Versiegen des Gasstroms - flüssige Ölrückstände, welche sich in der Gaszuführung und/oder der Gasabführung abgelagert haben, selbständig aufgrund der Schwerkraft in den Ansaugkanal und/oder einen Austrittskanal zurückfließen können. Dadurch wird verhindert, dass sich diese flüssigen Rückstände in der Messkammer ansammeln und dort die optische Messung stören.

Ferner können dabei auch Ölrückstände entgegen der Beschleunigungsrichtung und/oder entgegen dem zuvor erläuterten Anstieg aufgrund der Schwerkraft abfließen. Somit kann der Beschleunigungsabschnitt als auch der Anstieg problemlos eingesetzt werden, selbst wenn sich ab und an flüssige Ölrückstände in dem Ölnebeldetektor ansammeln.

Die Gaszuführung kann nun gerade so ausgestaltet sein, dass sie den zweiten, insbesondere restlichen, Teilgasstrom des Gasstroms entgegen der Beschleunigungsrichtung und/oder entgegen der Richtung des Anstiegs von dem Gasstrom abführt. Dazu kann die Gaszuführung insbesondere in einem spitzen Winkel in den Beschleunigungsabschnitt münden.

Es kann also alternativ oder ergänzend vorgesehen sein, dass die Gaszuführung unter einem spitzen Winkel bezogen auf die Beschleunigungsrichtung von dem Beschleunigungsabschnitt abzweigt. Dadurch können die kleineren Öltröpfchen bevorzugt gegenüber größeren Öltröpfchen von dem Beschleunigungsabschnitt in die Gaszuführung abgesondert werden, insbesondere wenn diese Öltröpfchen beim Abzweigen in die Gaszuführung eine Richtungsumkehr erfahren. Durch diese Maßnahmen wird die Effizienz der erfindungsgemäßen Separation der kleineren Öltröpfchen weiter verbessert.

Zu dem gleichen Zweck kann auch vorgesehen sein, dass sich ein Durchströmungsquerschnitt in Richtung der Gaszuführung im Bereich der Verzweigungsstelle vergrößert. Denn dadurch sinkt die Strömungsgeschwindigkeit stark ab, was dazu führt, dass sich die kleineren Öltröpfchen mit geringerer Trägheit homogen auch quer zur Beschleunigungsrichtung verteilen können. Dadurch gelangen die kleineren Öltröpfchen vermehrt in die Messkammer, während die schwereren Öltröpfchen aufgrund ihrer größeren Trägheit zunächst in der Beschleunigungsrichtung weiter fliegen und so vermehrt in den Bypass gelangen.

Gemäß einer weiteren vorteilhaften Ausgestaltung kann in Gasstromrichtung vor dem Beschleunigungsabschnitt eine Prallfläche ausgebildet sein. Eine solche Prallfläche kann bewirken, dass der Gasstrom bei Durchlaufen des Beschleunigungsabschnitts an der Prallfläche zunächst eine Richtungsumkehr erfährt. Bei einer solchen Richtungsumkehr können die Öltröpfchen somit für einen Moment lang eine Geschwindigkeit von Null aufweisen, um anschließend beschleunigt zu werden.

Darüber hinaus ist es vorteilhaft, wenn an einem Ende des Beschleunigungsabschnitts eine Umlenkfläche ausgebildet ist, die einen spitzen Winkel von weniger als 90° mit der Beschleunigungsrichtung ausbildet. Diese Umlenkfläche kann dann nämlich einen Fangtrichter ausbilden für den ersten Teilgasstrom. Mit anderen Worten kann somit der erste Teilgasstrom, welcher nach der erfolgten Absonderung der kleineren Öltröpfchen in die Gaszuleitung einen erhöhten Anteil an größeren Öltröpfchen aufweist, nach Passieren des Beschleunigungsabschnitts an der Umlenkfläche in den Bypass gelenkt werden, wobei die Umlenkfläche als Fangtrichter für schwerere Öltröpfchen fungiert.

Wie bereits erwähnt wurde, kann der Bypass, welcher den ersten Teilgasstrom abführt, in die Gasabführung münden, welche Gas aus der Messkammer abführt. Genauer kann der Bypass an einer Vereinigungsstelle so in die Gasabführung aus der Messkammer münden, dass der erste Teilgasstroms und der zweite, insbesondere restliche, Teilgasstrom an der Vereinigungsstelle wieder vereinigt werden.

Bei einer solchen Ausgestaltung ist es vorzuziehen, wenn ein Winkel zwischen einer mittleren Strömungsrichtung des ersten Teilgasstroms und einer mittleren Strömungsrichtung des zweiten, insbesondere restlichen, Teilgasstroms an der Vereinigungsstelle weniger als 60° beträgt. Denn dadurch können Gas-Verwirbelungen und damit ein Rückströmen von Gas entgegen der Hauptströmungsrichtung, insbesondere zurück in die Messkammer, vermieden werden, was die Messsicherheit erhöht.

Gemäß einer bevorzugten Ausgestaltung kann im Bereich der Vereinigungsstelle eine Venturi-Düse, beispielsweise mittels einer Querschnittsverengung im Bereich des Bypasses, ausgebildet sein. Denn dadurch kann der zweite Teilgasstrom zuverlässig, das heißt insbesondere unterbrechungsfrei, mittels einer von der Venturi-Düse erzeugten Druckdifferenz durch die Messkammer gefördert werden. Die Druckdifferenz wird dabei durch einen Druckabfall erzeugt, der dadurch entsteht, dass die Strömungsgeschwindigkeit des ersten Teilgasstroms an der Stelle der Querschnittsverengung ansteigt, wodurch der statische Druck an dieser Stelle abnimmt.

Um sicherzustellen, dass die schwereren Öltröpfchen vornehmlich in den Bypass und nicht in die Messkammer gelangen, ist es zudem empfehlenswert, wenn die Gaszuführung, welche in die Messkammer mündet, im Vergleich zu dem Bypass einen höheren Strömungswiderstand aufweist. Denn dadurch kann eine mittlere Strömungsgeschwindigkeit durch den Bypass eine mittlere Strömungsgeschwindigkeit durch die Messkammer übersteigen, was vorteilhaft ist für die beabsichtigte Separation, welche bevorzugt nur die kleineren Öltröpfchen in die Messkammer befördern soll. Der Bypass bildet somit für den ersten Teilgasstrom einen Hochgeschwindigkeitspass aus im Vergleich zum zweiten Teilgasstrom durch die Messkammer.

Dies kann beispielsweise besonders einfach bereits dadurch erreicht werden, dass der erste Teilgasstrom durch den Bypass eine kürzere Länge aufweist als der zweite Teilgasstrom durch die Gaszuführung, die Messkammer und die Gasabführung und zwar jeweils gemessen von der besagten Verzweigungsstelle aus bis zur Vereinigungsstelle. Beispielsweise kann die Länge des zweiten Teilgasstroms mindestens doppelt so lang sein, wie die des ersten Teilgasstroms.

Die Verwendung eines Bypasses, insbesondere in Kombination mit dem erwähnte Trägheitsabscheider und der Venturidüse, sorgt durch einen verringerten Schmutzeintrag in die Messkammer für zuverlässige Messergebnisse, verhindert Fehlalarme und vergrößert zudem die Wartungsintervalle.

Der Ölnebeldetektor weist bevorzugt eine (eigene) Gasfördervorrichtung auf. Dadurch können Gasproben aktiv von dem Ölnebeldetektor durch die Ansaugöffnung entnommen werden. Hierbei ist es vorteilhaft, wenn die Gasfördervorrichtung in der Einbausituation von außen zugänglich in dem Ölnebeldetektor angeordnet wird.

Eine solche Gasfördervorrichtung kann insbesondere als eine Gasförderpumpe zum Befördern des Gasstroms ausgestaltet sein, vorzugsweise in Form eines Radialgebläses. Hierbei ist es ferner bevorzugt, die Gasförderpumpe als eine Ansaug- und nicht etwa als eine Druckpumpe auszugestalten. Die Gasfördervorrichtung / Gasförderpumpe kann somit gasstromabwärts von der zuvor erläuterten Vereinigungsstelle platziert sein. Dadurch wird vermieden, dass eine ursprüngliche Größenverteilung der Öltröpfchen in dem Gasstrom durch Wechselwirkung mit einer Gasfördervorrichtung noch vor dem Eintritt in die optische Messkammer verfälscht wird, woraus sich falsche Messergebnisse ergeben könnten.

Zur Lösung der eingangs genannten Aufgabe sind ferner erfindungsgemäß bei einer Kolbenmaschine, insbesondere ausgestaltet als Verbrennungsmotor oder Kompressor, die Merkmale des Anspruchs 9 vorgesehen. Insbesondere wird somit erfindungsgemäß zur Lösung der Aufgabe bei einer Kolbenmaschine der eingangs genannten Art vorgeschlagen, dass der Ölnebeldetektor zur Entnahme von Gasproben aus dem Kurbelraum eingerichtet ist und gemäß einem der Ansprüche 1 bis 9 ausgestaltet ist. Für eine hohe Betriebssicherheit ist es hierbei empfehlenswert, dass der Ölnebeldetektor dicht in das Kurbelraumgehäuse der Kolbenmaschine eingesetzt ist.

Gemäß einer bevorzugten Ausgestaltung kann zur Entnahme der Gasproben eine Gasentnahmevorrichtung des Ölnebeldetektors, wie beispielsweise ein Ansaugrohr, durch das Kurbelraumgehäuse in den Kurbelraum geführt sein, wobei der Ölnebeldetektor, das heißt insbesondere die besagte Messkammer und/oder eine Gasfördervorrichtung des Ölnebeldetektors, von außen zugänglich ist.

Diese Ausgestaltung hat den Vorteil einer einfachen Wartung und Überprüfung des Ölnebeldetektors bei gleichzeitig einfacher Nachrüstbarkeit, da die Kolbenmaschine / der Verbrennungsmotor selbst über keine Vorrichtung verfügen muss, mit der der Ölnebel zum Ölnebeldetektor förderbar ist. Vielmehr genügt es, wenn die Kolbenmaschine / der Verbrennungsmotor eine, vorzugsweise einfach verschließbare, Öffnung aufweist, durch die Gasproben mit Hilfe des Ölnebeldetektors aus dem überwachten Innenraum entnommen werden können. Zudem kann die Messkammer oder die Gasfördervorrichtung gewartet werden, während der Ölnebeldetektor im Kurbelraumgehäuse eingebaut bleibt. Eine aufwändige Demontage des gesamten Ölnebeldetektors kann somit vermieden werden.

Zur Lösung der genannten Aufgabe sind erfindungsgemäß ferner die Merkmale des unabhängigen Verfahrensanspruchs 10 vorgesehen. Insbesondere wird somit erfindungsgemäß zur Lösung der Aufgabe bei einem Verfahren zum Betrieb eines Ölnebeldetektors, der erfindungsgemäß mit Merkmalen gemäß einem der Ansprüche 1 bis 9 ausgestaltet ist und eine Messkammer aufweist, die eine optische Messstrecke zur optischen Detektion und/oder Analyse eines Ölnebels ausbildet, vorgeschlagen, dass zur Detektion und/oder Analyse des

Ölnebels eine erste und eine zweite optische Messstrecke, die gegenläufig durch die Messkammer verlaufen, gleichzeitig oder alternierend für eine redundante Durchlichtmessung ausgewertet werden und dass zusätzlich mindestens eine Streulichtmessung unter Verwendung von Licht der ersten oder zweiten optischen Messstrecke zur Detektion und/oder Analyse des Ölnebels eingesetzt wird. Hierbei wird die Durchlichtmessung und die Streulichtmessung gleichzeitig ausgewertet.

Die Auswertung der Messsignale der jeweiligen Durchlichtbeziehungsweise Streulichtmessung kann dabei insbesondere von dem Ölnebeldetektor selbsttätig vorgenommen werden.

Von Vorteil ist dabei, dass die Sicherheit der optischen Charakterisierung einer Gasprobe, die mittels der optischen Messstrecken vermessen wird, erhöht werden kann, was insbesondere für die eingangs erläuterte Unterscheidung von Ölnebel und Wassernebel von Relevanz ist.

Es versteht sich, dass die Vorteile der Erfindung insbesondere dann voll ausgenutzt werden können, wenn bei diesem Verfahren ein Ölnebeldetektor verwendet wird und zwar mit Eigenschaften wie zuvor beschrieben und/oder gemäß einem der auf einen Ölnebeldetektor gerichteten Ansprüche.

Eine Weiterbildung dieses Verfahrens sieht vor, dass zur Detektion und/oder Analyse des Ölnebels mindestens eine dritte Messstrecke und eine vierte optische Messstrecke, die jeweils in einem Winkel zu der ersten oder zweiten Messstrecke verlaufen, gleichzeitig oder alternierend für eine mehrfach redundante Streulichtmessung ausgewertet werden. Hierbei ist es für eine effiziente Streulichtmessung vorzuziehen, wenn der besagte Winkel wenigstens 10°, bevorzugt wenigstens 30°, beträgt. Es können darüber hinaus noch weitere optische Messstrecken vorgesehen sein.

Bei einer solchen Ausgestaltung können demnach die erste und die zweite, als auch die dritte und vierte optische Messtrecke und gegebenenfalls weitere optische Messstrecken, innerhalb der Messkammer des Ölnebeldetektors verlaufen, sodass eine identische Gasprobe in der Messkammer mindestens zweifach redundant mittels insgesamt mindestens vier (insbesondere mittels mindestens sechs) unterschiedlichen Messstrecken optisch vermessen wird.

Zur Lösung der genannten Aufgabe sind erfindungsgemäß - gemäß dem zweiten Aspekt der Erfindung von möglicherweise eigenem erfinderischen Charakter - ferner die Merkmale des zweiten unabhängigen Verfahrensanspruchs vorgesehen. Insbesondere wird somit erfindungsgemäß zur Lösung der Aufgabe bei einem Verfahren zur Detektion und/oder Analyse potentiell explosiver Öl-Luftgemische in einem überwachten Raum wie eingangs beschrieben vorgeschlagen, dass eine Gasprobe mit Hilfe einer Gasfördervorrichtung des Ölnebeldetektors durch eine Ansaugöffnung des Ölnebeldetektors aus dem überwachten Raum, also beispielsweise aus einem Innenraum der zuvor beschriebenen Kolbenmaschine / des Verbrennungsmotors, entnommen wird und außerhalb des überwachten Raums / des Innenraums in der Messkammer optisch vermessen wird. Mit anderen Worten ist bei diesem Verfahren die Messkammer des Ölnebeldetektor somit außerhalb des überwachten Raums / des Innenraums angeordnet.

Dieses Verfahren bietet den großen Vorteil, dass die Sicherheit des Betriebs eines Verbrennungsmotors ohne größere konstruktive Veränderungen am Verbrennungsmotor selbst, nämlich durch einfaches modulares Nachrüsten eines erfindungsgemäßen Ölnebeldetektors an dem Verbrennungsmotor, gewährleistet werden kann. Insbesondere muss somit im Innenraum des Verbrennungsmotors keine aufwändige Anpassung erfolgen, sondern es genügt, eine bereits vorhandene Öffnung zur Entnahme einer Gasprobe aus dem Innenraum mit dem Ölnebeldetektor zu nutzen beziehungsweise hierfür anzupassen oder - falls keine vorhanden ist - diese auszubilden.

Es versteht sich, dass die besonderen Vorteile der Erfindung besonders dann voll genutzt werden können, wenn bei diesem Verfahren ein Ölnebeldetektor verwendet wird mit Eigenschaften wie zuvor beschrieben und/oder gemäß einem der auf einen Ölnebeldetektor gerichteten Ansprüche. Ferner kann auch dieses Verfahren mit Merkmalen des zuvor erläuterten Verfahrens in Bezug auf die Ausgestaltung der optischen Messung kombiniert werden, um die Vorteile der Erfindung zu realisieren und die beschriebenen Synergieeffekte zu nutzen.

Das Verfahren zur Detektion und/oder Analyse potentiell explosiver Öl-Luftgemische in einem überwachten Raum, insbesondere Innenraum, kann zudem weiter fortgebildet werden, indem die Gasprobe und ein daraus resultierender Gasstrom in dem Ölnebeldetektor in mindestens zwei Teilgasströme aufgeteilt wird, wobei diese Aufteilung / Separation insbesondere wie zuvor beschrieben geschehen kann. Demnach kann insbesondere ein erster Teilgasstrom durch einen Bypass unter Umgehung der Messkammer geführt werden, während ein zweiter, insbesondere restlicher, Teilgasstrom durch eine Gaszuführung in die Messkammer des Ölnebeldetektors geführt wird.

Hierbei ist es besonders bevorzugt, wenn mit Hilfe der Gaszuführung, die von einem in die Ansaugöffnung mündenden Ansaugkanal abzweigen kann, kleinere Öltröpfchen mit Durchmessern von weniger als 10 µm, die in der Gasprobe enthalten sind, von dem ersten Teilgasstrom separiert werden. Wie zuvor bereits detailliert beschrieben wurde, kann dadurch nämlich erreicht werden, dass ein relativer Anteil dieser kleineren Öltröpfchen in dem zweiten Teilgasstrom erhöht wird im Vergleich zu einem relativen Anteil dieser kleineren Öltröpfchen in der ursprünglich mit dem Ölnebeldetektor entnommenen Gasprobe beziehungsweise in dem daraus resultierenden Gasstrom. Diese Erhöhung führt zu einer zuverlässigeren optischen Vermessung des in der optischen Messkammer befindlichen Gasvolumens und damit zu einer Erhöhung der Messsicherheit.

Die Erfindung wird nun anhand eines Ausführungsbeispiels näher beschrieben, ist aber nicht auf dieses Ausführungsbeispiel beschränkt. Weitere Ausbildungen der Erfindung können aus der nachfolgenden Beschreibung eines bevorzugten Ausführungsbeispiels in Verbindung mit der allgemeinen Beschreibung, den Ansprüchen sowie den Zeichnungen gewonnen werden.

Bei der folgenden Beschreibung erhalten in ihrer Funktion übereinstimmende Elemente auch bei abweichender Gestaltung oder Formgebung übereinstimmende Bezugszahlen.

Es zeigt:
- Fig. 1: einen erfindungsgemäßen Ölnebeldetektor mit demontiertem Gehäuse, wobei die sonst im Gehäuse verbauten Leiterplatten und deren Befestigung nicht dargestellt sind,
- Fig. 2: den Ölnebeldetektor aus Figur 1 mit aufgesetztem Gehäuse,
- Fig. 3: eine Gasfördervorrichtung des Ölnebeldetektors aus Figur 1 in Form einer Ansaugpumpe in ausgebautem Zustand (vgl. Fig. 1, rechte Darstellung),
- Fig. 4: den Ölnebeldetektor aus Figur 2 eingesetzt in ein Kurbelraumgehäuse einer als Verbrennungsmotor ausgestalteten Kolbenmaschine,
- Fig. 5: eine Gasentnahmevorrichtung des Ölnebeldetektors aus Figur 1 und 2 betrachtet von schräg oben,
- Fig. 6: die Gasentnahmevorrichtung aus Figur 5 betrachtet von schräg unten,
- Fig. 7: eine frontale Ansicht von vorne auf die Gasentnahmevorrichtung aus Figur 5,
- Fig. 8: einen Längsschnitt durch die Gasentnahmevorrichtung aus Figur 5,
- Fig. 9: eine Ansicht von schräg vorne auf die Vorderseite des Ölnebeldetektors aus Figur 1 mit abgesetztem Gehäuse, ohne Leiterplatten und bei abgenommener Gasentnahmevorrichtung und abgenommenem Servicedeckel,
- Fig. 10: eine Ansicht von schräg hinten auf die Rückseite des Ölnebeldetektors aus Figur 9,
- Fig. 11: eine Draufsicht auf die Vorderseite des Ölnebeldetektors bei abgenommenem Deckel,
- Fig. 12: eine Seitenansicht von unten auf den Ölnebeldetektor mit abgesetztem Gehäuse,
- Fig. 13: eine Draufsicht auf die Rückseite des Ölnebeldetektors, mit einer Detailansicht in einer tieferen Ebene (schraffierte Flächen),
- Fig. 14: die Detailansicht aus Figur 13 in einer vergrößerten Darstellung mit einer Illustration verschiedener Teilgasströme und einer weiteren vergrößerten Detailansicht der Gasströmungen im Bereich des Bypasses und der Messkammer.
- Fig. 15: den Ölnebeldetektor aus Figur 2 mit abgenommenem Spannelement,
- Fig. 16: eine Seitenansicht des Ölnebeldetektors aus Figur 2 mit einer teilweisen Schnittansicht im Bereich der Gasfördervorrichtung,
- Fig. 17: die Gasfördervorrichtung aus Figur 3 in einer Schnittdarstellung mit einer gegenüber Figur 3 um 90° um eine Längsachse gedrehten Schnittebene,
- Fig. 18: die Gasfördervorrichtung aus Figur 3 und Figur 17 in einer perspektivischen Ansicht von vorne und
- Fig. 19: eine weitere mögliche Ausgestaltung einer Gasentnahmevorrichtung des Ölnebeldetektors.

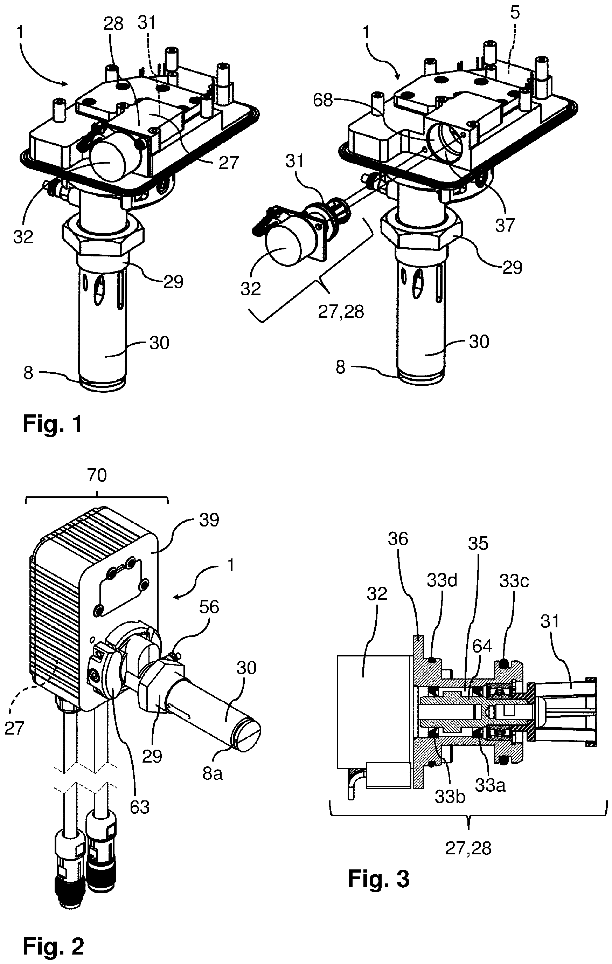

Die Figur 2 zeigt einen erfindungsgemäßen Ölnebeldetektor mit aufgesetztem Gehäuse, während Figur 1 den Ölnebeldetektor mit abgesetztem Gehäuse und ohne Leiterplatte(n) zeigt, einmal mit eingesetzter Gasfördervorrichtung 27 (links) und einmal mit ausgebauter Gasfördervorrichtung 27 (rechts).

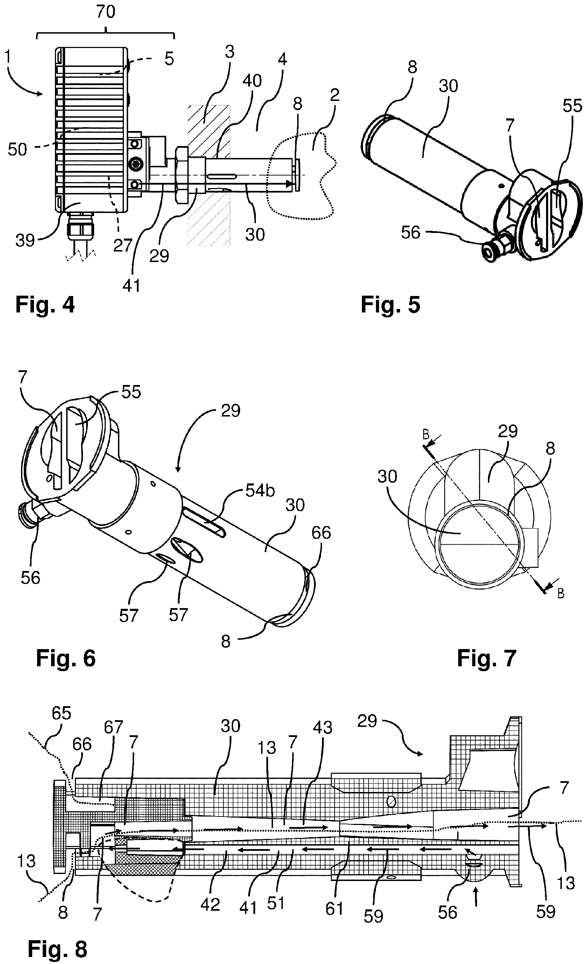

Wie in Figur 4 illustriert ist, ist der Ölnebeldetektor 1 zum Einsetzen in ein Kurbelraumgehäuse 3 einer (in Figur 4 nicht näher illustrierten) als Verbrennungsmotor ausgestalteten Kolbenmaschine ausgelegt. Hierzu weist der Ölnebeldetektor 1 eine Gasentnahmevorrichtung 29 an einem Messteil 70 auf (Vgl. die Figur 4), die durch das Kurbelraumgehäuse 3 in den Kurbelraum 4 des Verbrennungsmotors geführt werden kann, um dort eine Gasprobe entnehmen zu können. Der Messteil 70 verbleibt hierbei außerhalb des Kurbelraums 4, der mit dem Ölnebeldetektor 1 überwacht wird.

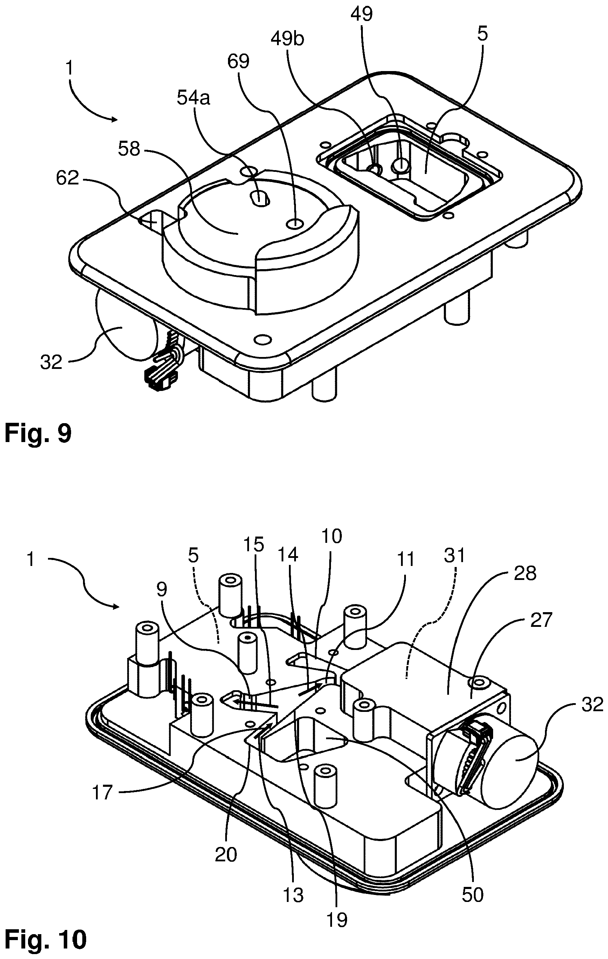

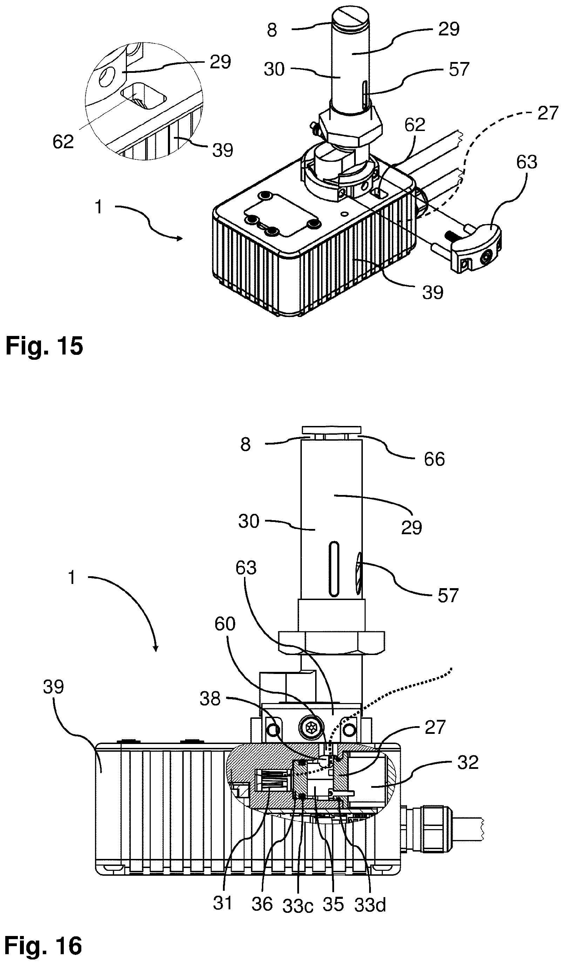

Wie in der Figur 15 illustriert, ist die Gasentnahmevorrichtung 29 abnehmbar ausgestaltet, um diese reinigen zu können. Mittels einer an dem Gehäuse 39 des Ölnebeldetektors ausgebildeten Schnittstelle 58 (Vgl. die Figur 9) und eines separaten Spannelements 63 (Vgl. die Figuren 2 und 15) lässt sich die Gasentnahmevorrichtung 29 an dem Ölnebeldetektor 1 sicher und abdichtend montieren.

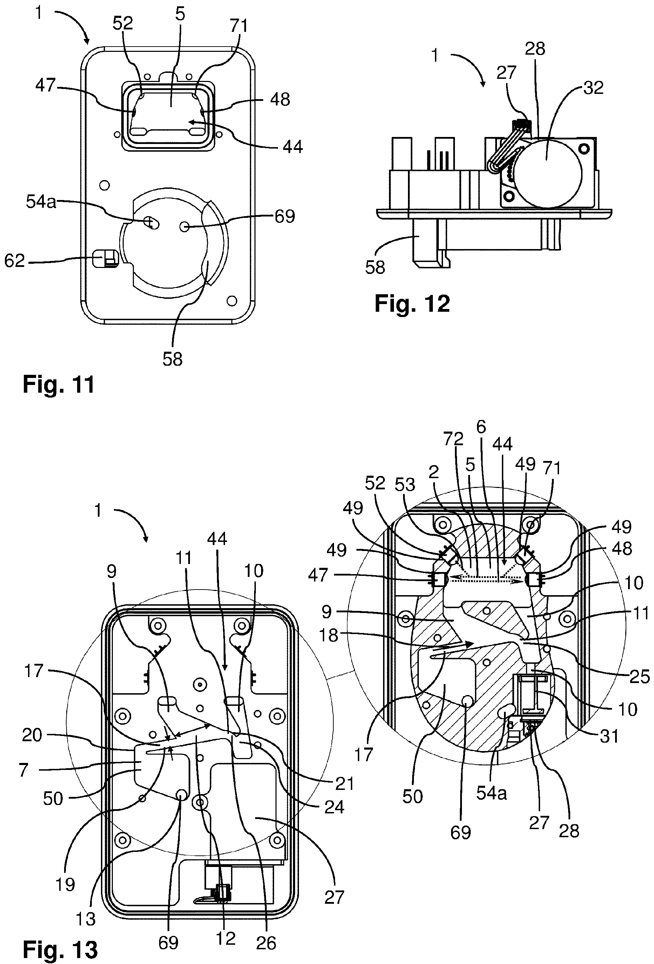

Vorteilhaft an diesem Ansatz ist insbesondere, dass der Ölnebeldetektor 1, das heißt insbesondere die in Figur 11 gezeigte Messkammer 5 und die in Figur 1 und 3 gezeigte Gasfördervorrichtung 27 des Ölnebeldetektors 1, von außen zugänglich sind. Außerdem kann der Ölnebeldetektor 1 abgenommen werden, während die Gasentnahmevorrichtung 29 in das Kurbelraumgehäuse 3 eingesetzt bleibt.

Die Gasfördervorrichtung 27 des Ölnebeldetektors 1, die in der in Figur 4 gezeigten Einbausituation außen in Bezug auf den Kurbelraum 4 angeordnet ist, dient der Entnahme von Gasproben aus dem Kurbelraum 4. Mit Hilfe der Gasfördervorrichtung 27, die als eine motorgetriebene Gasförderpumpe 28 ausgestaltet ist, wie in Figur 3 zu erkennen ist, kann der Ölnebeldetektor 1 somit eine Gasprobe je nach Messbedarf aktiv aus dem Kurbelraum 4 entnehmen. Dabei wird die Gasprobe durch die in Figur 4 zu erkennende ringförmige Ansaugöffnung 8 der Gasentnahmevorrichtung 29 angesaugt und durch einen in einem Ansaugrohr 30 der Gasentnahmevorrichtung 29 ausgestalteten Ansaugkanal 7, der in Figur 8 genauer illustriert ist, aus dem Kurbelraum 4 durch das Kurbelraumgehäuse 3 hindurch bis in eine außerhalb des Kurbelraums 4 angeordnete Messkammer 5 des Ölnebeldetektors 1 gefördert, die in Figur 9 zu sehen ist.

Ein Rückführstrom 65 wird durch einen Rückführkanal 67 über eine Rückführöffnung 66 in den Kurbelraum 4 rückgeführt. Hierbei ist die Rückführöffnung 66 von der Ansaugöffnung 8 abgewandt, um einen fluidischen Kurzschluss, also das unmittelbare Ansaugen des Gases, das aus der Rückführöffnung 66 austritt, zu vermeiden.

Wie die Figuren 1, 3 und 13 zeigen, weist die Gasförderpumpe 28 zum Befördern des Gasstroms 13 eine Lüfterflügelanordnung 31 auf, die von einem elektrischen Antriebsmotor 32 angetrieben wird. Die Gasförderpumpe 28 bildet somit zusammen mit einer Wandung 68 der Aufnahme 37 ein Radialgebläse. Die Gasförderpumpe 28 ist ferner als Ansaugpumpe ausgestaltet, da sie gasstromabwärts von der Vereinigungsstelle 24 platziert ist und somit den Gasstrom 13 durch die Gasentnahmevorrichtung 29 aus dem Kurbelraum 4 ansaugt. Dies ist vorteilhaft, da der Gasstrom 13 somit erst nach Passieren der Messkammer 5 in Kontakt mit der Lüfterflügelanordnung 31 gerät. Dadurch kann vermieden werden, dass die Größenverteilung der Öltröpfchen durch eine Wechselwirkung mit der rotierenden Lüfterflügelanordnung 31 verfälscht wird.

Mit diesem Ansatz wird ermöglicht, einen in dem Kurbelraum 4 entstehenden Ölnebel 2, also ein Aerosol aus feinsten Öltröpfchen, mit dem Ölnebeldetektor 1 optisch zu detektieren. Denn der Ölnebel 2 kann als Teil der Gasprobe mit der Gasentnahmevorrichtung 29 in die Messkammer 5 befördert werden und dort optisch vermessen werden. Somit kann mit dem Ölnebeldetektor 1 die Entstehung von feinem Ölnebel 2 in dem Kurbelraum 4 überwacht werden.

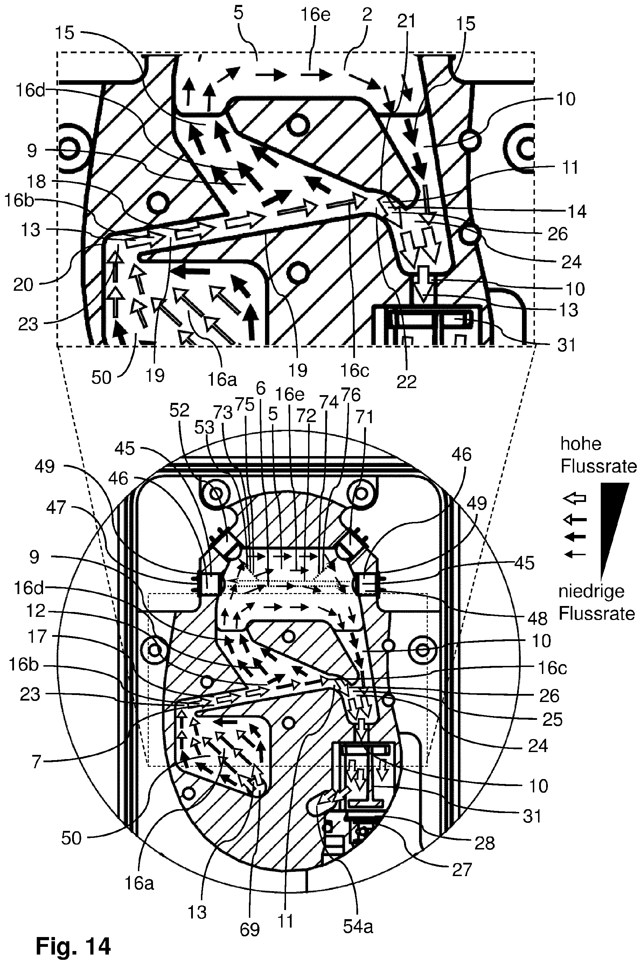

Zu diesem Zweck ist in der Messkammer 5 des Ölnebeldetektors 1, wie in der Detailansicht der Figur 13 gut zu erkennen ist, eine optische Messstrecke 6 zur optischen Detektion des in die Messkammer 5 geförderten Ölnebels 2 ausgebildet. Genauer weist die Messkammer 5 eine optische Messanordnung 44 auf. Diese umfasst Lichtquellen 45 und Lichtdetektoren 46 und ist zur Durchführung einer Durchlichtmessung und einer Streulichtmessung an der in der Messkammer 5 befindlichen Gasprobe eingerichtet. Denn wie unschwer in der Detailansicht der Figur 13 zu erkennen ist, ist eine erste optische Messtrecke 6 für eine erste Durchlichtmessung ausgebildet, sowie eine zweite optische Messtrecke 72 für eine zweite redundante Durchlichtmessung desselben Gasvolumens. Daneben sind, wie die Figur 14 illustriert, noch vier weitere optische Messstrecken 73, 74, 75 und 76 ausgebildet, die für eine jeweilige Streulichtmessung vorgesehen sind. Alle diese sechs Messstrecken 6,72,73,74,75 und 76 können gleichzeitig ausgewertet werden.

Zur Durchlichtmessung umfasst die optische Messanordnung 44 ein erstes Funktionspaar 47 aus einer Lichtquelle 45 und einem Lichtdetektor 46, sowie ein zweites solches Funktionspaar 48, welches analog ausgebildet ist (Vgl. Figur 13 und 14). Die Lichtquellen 45 und Lichtdetektoren 46 der beiden Funktionspaare 47, 48 sind dabei jeweils so ausgerichtet, dass die Lichtquelle 45 des ersten Funktionspaars 47 mit dem Lichtdetektor 46 des zweiten Funktionspaars 48 die erste optische Messtrecke 6 ausbildet, während die Lichtquelle 45 des zweiten Funktionspaars 48 mit dem Lichtdetektor 46 des ersten Funktionspaars 47 die zweite optische Messtrecke 72 ausbildet. Hierbei verlaufen die beiden optischen Messstrecken 6 und 72 gerade gegensinnig und legen dieselbe Messtrecke durch die Messkammer 5 zurück. Mit anderen Worten bildet die erste und die zweite optische Messtrecke 6 und 72, beziehungsweise das erste Funktionspaar 47 und das zweite Funktionspaar 48, somit ein redundantes bidirektionales Messpaar aus.

Die erste und die zweite optische Messstrecke 6, 72 verlaufen dabei in zueinander entgegengesetzten Richtungen und parallel zueinander, wie man in der Ansicht der Figur 14 gut erkennt. In Figur 13 und 14 ist darüber hinaus zu erkennen, dass die beiden Funktionspaare 47, 48 jeweils baulich als eine Einheit ausgestaltet sind. Dabei ist jede der Einheiten in eine jeweilige Aufnahme 49 der Messkammer 5 eingesetzt. In Figur 14 ist zudem gut zu erkennen, dass sich die beiden Aufnahmen 49 der Funktionspaare 47, 48 gerade so gegenüberliegen, dass die erste und zweite optische Messstrecke 6, 72 mittig durch die Messkammer 5 verlaufen.

Mittels eines in Figur 14 illustrierten weiteren Lichtdetektors 52, der in Bezug auf die erste optische Messstrecke 6 eine Sichtlinie 53 in einem Winkel von mehr als 10° ausbildet, kann Streulicht, genauer Licht welches die Lichtquelle 45 des linken Funktionspaars 47 aussendet und welches an Öltröpfchen in der Messkammer 5 zurückgestreut wird, detektieren. Daneben ist noch ein zweiter Streulicht-Detektor 71 ausgebildet. Dieser bildet ebenfalls eine Sichtlinie aus, die einen Winkel von mehr als 10° mit der ersten optischen Messstrecke 6 ausbildet. Allerdings empfängt dieser zweite weitere Lichtdetektor 71 vorwärts gestreutes Licht, welches ursprünglich von der Lichtquelle 45 des linken Funktionspaars 47 ausgesendet wurde.

In Bezug auf die Lichtquelle 45 des zweiten rechten Funktionspaars 48 empfängt der erste weitere Lichtdetektor 52 vorwärts gestreutes Licht (Messstrecke 73) und der zweite weitere Lichtdetektor 71 rückgestreutes Licht (Messstrecke 74). Umgekehrt empfängt der Lichtdetektor 52 rückwärts gestreutes Licht der linken Lichtquelle 45 (Messstrecke 75) und der Lichtdetektor 71 vorwärts gestreutes Licht der linken Lichtquelle 45 (Messstrecke 76). Mit anderen Worten bilden somit die Lichtdetektoren 52 und 71 ein redundantes Messpaar für (mehrfach) redundante Streulichtmessungen mit vorwärts und rückwärts gestreutem Licht.

Der zweite weitere Lichtdetektor 71 ist somit zur Durchführung einer zweiten Streulichtmessung vorgesehen, nämlich unter Verwendung von Licht, welches die Lichtquelle 45 des zweiten Funktionspaars 48 aussendet. Diese zweite Streulichtmessung ist redundant zu der mittels des ersten weiteren Lichtdetektors 52 möglichen ersten Streulichtmessung unter Verwendung von Licht, welches die Lichtquelle 45 des ersten Funktionspaars 47 aussendet. Beispielsweise kann in Bezug auf von der linken Lichtquelle 45 ausgesandtes Licht der Detektor 71 vorwärts gestreutes Licht messen und der weitere Detektor 52 kann vorwärts gestreutes Licht der rechten Lichtquelle 45 messen.

Um von der von der Messanordnung 44 bereit gestellten Redundanz profitieren zu können verfügt der Ölnebeldetektor 1 über eine Berechnungseinheit, die dazu eingerichtet ist, zwei unabhängige Durchlichtmessungen mittels der ersten und zweiten optischen Messstrecke 6, 72, insbesondere gleichzeitig, auszuwerten. Dabei wird konstant eine Dioden-spezifische Verschmutzung ermittelt und bei späteren Berechnungen berücksichtigt. Durch diese Funktion kann das Wartungs/Reinigungsintervall vergrößert werden.

Alternativ oder bei zu großen Verschmutzungen verwirft die Berechnungseinheit Messergebnisse, wenn die Messsignale aus der ersten und zweiten optischen Messstrecke 6, 72 zu stark voneinander abweichen.

Mit anderen Worten führt die Berechnungseinheit somit eine Plausibilitätsprüfung der Durchlichtmessung anhand von mit der zweiten optischen Messstrecke 72 gewonnenen Messdaten durch, und zwar in Bezug auf Messdaten, die mit der ersten optischen Messstrecke 6 gewonnen wurden. Zusätzlich kann die Berechnungseinheit in analoger Weise auch eine Plausibilitätsprüfung einer der Streulichtmessungen durchführen, zum Beispiel anhand von mit der zweiten Streulichtmessung 73/75 gewonnenen Messdaten, und zwar in Bezug auf Messdaten, die mit der ersten Streulichtmessung 74/76 gewonnen wurden (entweder für vorwärts oder rückwärts gestreutes Licht).

Die Berechnungseinheit, die in Form einer Elektronik-Einheit ausgestaltet ist, ist ferner dazu eingerichtet, eine Lichtabsorption auf Basis der beiden redundanten Durchlichtmessungen mit Hilfe der optischen Messstrecken 6 und 72 sowie eine Streulichtintensität auf Basis der mehrfach redundanten Streulichtmessungen (mittels vorwärts und rückwärts gestreutem Licht) mit Hilfe der optischen Messstrecken 73 und 74 quantitativ zu bestimmen. Aus solchen quantitativen Messungen lassen sich Rückschlüsse ziehen über die Konzentration des Ölnebels 2 in der Messkammer 5. Insbesondere kann dadurch zumindest indirekt ermittelt werden, ob der Ölnebel 2 Öltröpfchen aufweist, die ein potentiell explosives Öl-Luft-Gemisch in dem überwachten Raum 4 bilden können, auch wenn die genaue Größe der Öltröpfchen unbekannt bleibt.

Die Berechnungseinheit ist darüber hinaus in der Lage, anhand der bestimmten Lichtabsorption und der bestimmten Streulichtintensität Messsignale, die einem Wasseranteil der Gasprobe in der Messkammer 5 zuordenbar sind, von Messsignalen zu unterscheiden, die einem Anteil eines Aerosols von Öltröpfchen, insbesondere von weniger als 10 µm Durchmesser, der Gasprobe zuordenbar sind. Hierzu verwendet die Berechnungseinheit gerade die bestimmte Streulichtintensität zur Differenzierung der beiden Messsignale, die vom Wasseranteil beziehungsweise vom Öl-Aerosol-Anteil erzeugt werden.

Um einen sicheren Betrieb des Verbrennungsmotors (als ein Beispiel einer mit dem Ölnebeldetektor 1 überwachbaren Kolbenmaschine) zu gewährleisten, gibt die Berechnungseinheit ein entsprechendes Warnsignal aus, wenn sie auf Basis der bestimmten Lichtabsorption und/oder der bestimmten Streulichtintensität auf die Anwesenheit des besagten Aerosols von Öltröpfchen schließt. In einem solchen Fall kann also der Betrieb des Verbrennungsmotors eingestellt werden.

Die Berechnungseinheit realisiert somit ein erfindungsgemäßes Verfahren zur Detektion des Ölnebels 2 in der Messkammer 5, bei dem die beiden Messtrecken 6 und 72 gleichzeitig oder alternierend für eine redundante Durchlichtmessung ausgewertet werden, während zusätzlich eine weitere Streulichtmessung, entweder unter Verwendung von Licht der ersten Messtrecke 6 oder 72 und eines oder beiden der weiteren Lichtdetektoren 52 und 71, erfolgt und wobei die Detektion des Ölnebels 2 auf der Durchlichtmessung und der Streulichtmessung beruht.

Hierbei wird, wie bereits beschrieben, die Dioden-spezifische Verschmutzung erkannt und bei der Messung berücksichtigt. Das verlängert die Wartungsintervalle, ergibt eine höhere Genauigkeit und/oder unterbindet Fehlalarme.

Die Berechnungseinheit setzt bei diesem Verfahren ferner die beiden weiteren Lichtdetektoren 52 und 71 gleichzeitig oder alternierend ein, um die Streulichtmessung redundant auszugestalten. Hierfür werden die weiteren optischen Messtrecken 73 und 74 eingesetzt, die in Figur 14 illustriert sind und die jeweils in einem Winkel von mehr als 10° zur ersten bzw. zweiten Messtrecke 6 / 72 verlaufen.

Der Ölnebeldetektor 1 weist darüber hinaus noch weitere Merkmale auf, die möglicherweise von eigener erfinderischer Qualität sind. Denn um eine möglichst genaue Vermessung der kritischen kleinen Öltröpfchen mit Durchmessern von weniger als 10 µm in der Messkammer 5 zu ermöglichen, müssen diese von größeren Öltropfen, die sich ebenfalls in der Gasprobe befinden können oder sich aufgrund von Kondensation oder Agglomerationsprozessen erst beim Transport der Gasprobe durch den Ölnebeldetektor 1 bilden, separiert werden.

Zu diesem Zweck ist vorgesehen, wie anschaulich anhand der Figur 13 und 14 nachvollzogen werden kann, einen Bypass 11 auszubilden, der einen ersten Teil des über die Gasentnahmevorrichtung 29 in den Ölnebeldetektor 1 einströmenden Gasstroms 13 als einen ersten Teilgasstrom 14 an der Messkammer 5 vorbeiführt. Dieser Teil der Gasprobe beziehungsweise des Gasstroms 13 gelangt somit erst gar nicht in die Messkammer 5 und stört dort die optische Messung nicht.

Eine genauere Betrachtung der Figuren 13 und 14 zeigt, dass die Messkammer 5 mittels einer Gaszuführung 9 mit dem zuvor bereits erläuterten Ansaugkanal 7 verbunden ist. Der Ansaugkanal 7 erstreckt sich dabei von der im Kurbelraum 4 liegenden Ansaugöffnung 8 der Gasentnahmevorrichtung 29 (Vgl. Figur 4) entlang des Ansaugrohrs 30 (Vgl. Figur 8) durch die in Figur 13 erkennbare zweite Ansaugöffnung 69 in eine Vorkammer 50 und von dort bis zur Gaszuführung 9 und zum Bypass 11.

Die Vorkammer 50 dient dabei als eine Art Beruhigungszone, in welcher Turbulenzen in dem Gasstrom 13 abgebaut werden können. Im Ergebnis findet in der Vorkammer 50 eine erste Abscheidung von größeren Ölpartikeln ab. Diese können dann über die Ansaugöffnung 69, der Schwerkraft folgend, zurück in die Gasentnahmevorrichtung 29 und von dort über die in Figur 6 erkennbaren Ausflussöffnungen 57 in den (mit dem Ölnebeldetektor 1 überwachten) Innenraum 4 zurückfließen. Verantwortlich für diese Beruhigung des Gasstroms 13 ist der - im Vergleich zu dem sich anschließenden Abschnitt des Ansaugkanals 7 - vergrößerte Durchflussquerschnitt der Vorkammer 50.

Ferner ist eine Gasabführung 10 ausgebildet als ein Kanal zum Abführen von Gas aus der Messkammer 5. Die Gasabführung 10 erstreckt sich dabei von der Messkammer 5 ausgehend bis zur Gasfördervorrichtung 27 (Vgl. Figur 14). Von dort wird der wiedervereinigte Gasstrom 13 weiter durch die in Figur 11, 13 und 14 erkennbare erste Austrittsöffnung 54a in einen Austrittskanal 55 der Gasentnahmevorrichtung 29 (Vgl. die Figuren 5 und 6) und von dort weiter bis zu einer - in der Einbausituation - im Kurbelraum 4 angeordneten Öffnungen 54b und 66, die in Figur 6 gut zu erkennen sind, gefördert. Der Austrittskanal 55 bildet zudem noch zwei weitere Ausflüsse 57 aus, durch die Öl aus dem Ölnebeldetektor 1 in den Kurbelraum 4 zurückfließen kann (Vgl. Figur 6).

Mit Hilfe des Ölnebeldetektors 1 ist somit ein Gasflusskreislauf gebildet, welcher - angetrieben von der Gasfördervorrichtung 27 des Ölnebeldetektors 1 - vom Kurbelraum 4 durch die Gasentnahmevorrichtung 29 in den Ölnebeldetektor 1 führt und anschließend erneut durch die Gasentnahmevorrichtung 29 - im Beispiel über einen separaten Rückführkanal 67 - zurück in den Kurbelraum 4.

Der vorgesehene Bypass 11 dient dabei zum Abführen von Gas aus dem Ansaugkanal 7 unter Umgehung der Messkammer 5 in den Austrittskanal 55 und von dort zurück in den Kurbelraum 4. Besonders einfach lässt sich dies realisieren, wenn - wie im Ausführungsbeispiel der Figuren gezeigt - der Bypass 11 die Gaszuführung 9 mit der Gasabführung 10 verbindet.

Wie aus der Illustration der verschiedenen Gasflüsse innerhalb des Ölnebeldetektors 1 gemäß Figur 14 zu erkennen ist, wird der durch die Gasentnahmevorrichtung 29 einströmende Gasstrom 13 somit in den ersten Teilgasstrom 14, welcher durch den Bypass 11 strömt, und einen restlichen Teilgasstrom 15, welcher durch die Gaszuführung 9 in die Messkammer 5 und von dort in die Gasabführung 10 strömt, aufgeteilt. Kurz nach dem Bypass 11 werden diese beiden Teilgasströme 14, 15 dann an der in Figur 13 illustrierten Vereinigungsstelle 24 wieder zusammengeführt, bevor sie als ein gemeinsamer wiedervereinigter Gasstrom 13 von der Gasfördervorrichtung 27 zurück in die Gasentnahmevorrichtung 29 befördert werden. Um bei dieser Vereinigung möglichst wenig Turbulenzen entstehen zu lassen, kann der Winkel zwischen der mittleren Strömungsrichtung des ersten Teilgasstroms 14 und des zweiten Teilgasstroms 15 an der Vereinigungsstelle 24 beispielsweise weniger als 60° betragen.

Der Ansaugkanal 7 verzweigt sich an der in Figur 13 und 14 illustrierten Verzweigungsstelle 12 somit in die Gaszuführung 9 und den Bypass 11. Dadurch ist der erste Teilgasstrom 14, welcher durch die Ansaugöffnung 8 und den Ansaugkanal 7 strömt, durch den Bypass 11 hindurch und an der Messkammer 5 vorbei in die Gasabführung 10 geleitet.

Der zweite, restliche Teilgasstrom 15 des durch den Ansaugkanal 7 strömenden Gasstroms 13 ist hingegen durch die Gaszuführung 9 in die Messkammer 5 geleitet.

Die Aufteilung des einströmenden Gasstroms 13 in die beiden Teilgasströme 14 und 15 verfolgt das Ziel, größere Öltröpfchen in den Bypass 11 abzusondern und vornehmlich kleinere Öltröpfchen, die für die Explosionsgefahr relevant sind, in die Messkammer 5 zu leiten, damit diese dort - ungestört von den größeren Öltröpfchen - präzise vermessen werden können.

Zu diesem Zweck zweigt die Gaszuführung 9 an der Verzweigungsstelle 12 von dem Ansaugkanal 7, bezogen auf die in Figur 4 gezeigte Einbausituation, nach oben ab. Dadurch müssen Öltröpfchen, die in die Messkammer 5 gelangen, somit entgegen der Schwerkraft wandern, was für kleinere Öltröpfchen wahrscheinlicher ist als für größere Öltröpfchen.

Das Abscheiden der großen Öltröpfchen hat weiter den Vorteil, dass die Verschmutzung der Messkammer/ der Dioden vermindert wird, was die Wartungs-/Reinigungsintervalle erheblich streckt. In bestimmten Anwendungen kann so ein zuverlässiger Betrieb des Ölnebeldetektors 1 über Jahre hinweg gewährleistet werden.

Mit anderen Worten führt somit in der Einbausituation die Gaszuführung 9 den zweiten Teilgasstrom 15 entgegen der Schwerkraft und - bei genauer Betrachtung der Figur 14 - auch entgegen einer Strömungsrichtung 16c des ersten Teilgasstroms 14 von dem Ansaugkanal 7 ab. Dadurch wird erreicht, dass der erste Teilgasstrom 14 überwiegend größere Öltröpfchen, insbesondere von mindestens 10 µm Durchmesser, aufweist, während der zweite Teilgasstrom 15 überwiegend kleinere Öltröpfchen von weniger als 10 µm Durchmesser aufweist.

Um die Separation der größeren Öltröpfchen von den kleineren Öltröpfchen zu verbessern ist zusätzlich in dem Ansaugkanal 7 noch vor der Verzweigungsstelle 12 ein Beschleunigungsabschnitt 17 ausgebildet (Vgl. Figur 10). Wie die Figur 14 anhand der unterschiedlich stark ausgeprägten Pfeile zeigt, die unterschiedlich hohe Strömungsgeschwindigkeiten repräsentieren, dient der Beschleunigungsabschnitt 17 dem Beschleunigen des Gasstroms 13 entlang der in Figur 13 illustrierten Beschleunigungsrichtung 18, nachdem dieser die Vorkammer 50 passiert hat. Dadurch wird insbesondere auch der erste Teil des Gasstroms 13, der schließlich in den Bypass 11 gelangt, beschleunigt.

Durch die Beschleunigung wird erreicht, dass gerade die größeren Öltröpfchen eine hohe kinetische Energie erreichen, sodass diese zunächst geradlinig weiter fliegen, bis sie auf die in Figur 14 illustrierte Umlenkfläche 21 treffen. Die Umlenkfläche 21 bildet dabei eine Art Fangtrichter 22 aus, der die schweren Öltröpfchen einsammelt und in den Bypass 11 leitet. Zu diesem Zweck nimmt die Umlenkfläche 21 einen spitzen Winkel von weniger als 90° mit der Beschleunigungsrichtung 18 ein.

Durch den Beschleunigungsabschnitt 17 und den Bypass 11 wird somit ein Trägheitsabscheider ausgebildet, welcher schwere Öltröpfchen oder sonstigen Schmutz in den Bypass 11 abscheidet und so verhindert, dass diese die Messkammer verschmutzen.

Die kleinen und damit leichteren Öltröpfchen können ihre Richtung hingegen leichter ändern, da sie eine vergleichsweise geringe kinetische Energie aufweisen, und werden daher bevorzugt noch vor der Umlenkfläche 21 in die Gaszuführung 9 abgesondert und gelangen so in die Messkammer 5.

Gut zu erkennen in den Figuren 10 und 13 ist, dass der Beschleunigungsabschnitt 17 in der Einbausituation in Richtung des Gasstroms 13 einen Anstieg 19 ausbildet. Somit sammeln sich die schwereren Öltröpfchen im Bereich der Verzweigungsstelle 12 bevorzugt am unteren Kanalboden des Ansaugkanals 7 an und wandern entlang des Anstiegs 19 bis zum Bypass 11.

Darüber hinaus ist es für eine effiziente Separation der kleineren Öltröpfchen vorteilhaft, wenn - wie in Figur 13 gezeigt, die Gaszuführung 9 an der Verzweigungsstelle 12 von dem Ansaugkanal 7, bezogen auf die in Figur 4 illustrierte Einbausituation, nach oben abzweigt. Zum Verständnis sei hier nochmals erwähnt, dass die Messkammer 5 in der Einbausituation am höchsten liegt (Vgl. Figur 4), sodass die Gaszuführung 9 und die Gasabführung 10 von unten in die Messkammer 5 münden (Vgl. Figur 13), wie ein Vergleich der Figuren 4, 11 und 13 veranschaulicht. Dies hat unter anderem den Vorteil, dass bei Versiegen des Gasstroms 13 flüssige Ölrückstände, die sich in der Gaszuführung 9 und/oder der Gasabführung 10 ablagern, selbständig aufgrund der Schwerkraft in den Ansaugkanal 7 und/oder den Austrittskanal 55 zurückfließen können und sich somit nicht in der Messkammer 5 ansammeln. Bei der in Figur 13 gezeigten Ausführungsform fließen dabei die Ölrückstände aufgrund der Schwerkraft entgegen der illustrierten Beschleunigungsrichtung 18 und entgegen dem Anstieg 19 ab.

Wie in den Figuren 11, 13 und 14 zu sehen ist, führt die Gaszuführung 9 den zweiten Teilgasstrom 15 somit entgegen der Beschleunigungsrichtung 18 und auch entgegen der Richtung des Anstiegs 19 von dem durch den Ansaugkanal 7 strömenden Gasstrom 13 ab. Hier ist die Gaszuführung 9 unter einem spitzen Winkel bezogen auf die Beschleunigungsrichtung 18 von dem Beschleunigungsabschnitt 17 abgezweigt. Wie die Strömungspfeile in Figur 14 illustrieren, führt dies dazu, dass die Öltröpfchen, die dem zweiten Teilgasstrom 15 folgen, bezogen auf die ursprüngliche Beschleunigungsrichtung 18 eine Richtungsumkehr erfahren. Diese Richtungsumkehr ist für schwerere Öltröpfchen mit hoher kinetischer Energie jedoch gerade sehr unwahrscheinlich, sodass diese weniger häufig oder gar nicht in die Gaszuführung 9 gelangen.

Ferner ist in den Figuren 11, 13 und 14 zu erkennen, dass sich ein Durchströmungsquerschnitt in Richtung der Gaszuführung 9 im Bereich der Verzweigungsstelle 12 vergrößert, im Vergleich zum dem Durchströmungsquerschnitt des angrenzenden Ansaugkanals 7, wie anhand der beiden Doppelpfeile in Figur 13 illustriert ist.

In Figur 11 und 13 ist auch zu sehen, dass in Gasstromrichtung 16a vor dem Beschleunigungsabschnitt 17 eine Prallfläche 20 ausgebildet ist, und zwar derart, dass der durch die Vorkammer 50 strömende Gasstrom 13 vor Durchlaufen des Beschleunigungsabschnitts 17 an der Prallfläche 20 eine Richtungsumkehr 23 erfährt. Dadurch erfahren alle Öltröpfchen eine Richtungsänderung, um anschließend in Richtung der Beschleunigungsrichtung 18 beschleunigt zu werden.

Die Förderung des zweiten Teilgasstroms 15 durch die Messkammer 5 geschieht mit Hilfe einer Venturi-Düse 25, die mittels einer Querschnittsverengung 26 im Bereich des Bypasses 11, ausgebildet ist, wie in Figur 13 und 14 illustriert. Dadurch wird der zweite Teilgasstrom 15 mittels eines von der Venturi-Düse 25 erzeugten Druckdifferenz durch die Messkammer 5 gefördert, denn wie in Figur 14 anhand der Strömungspfeile zu erkennen ist, ist die Gasströmungsgeschwindigkeit im Bereich der Querschnittsverengung 26 am höchsten.

Um diese hohe Strömungsgeschwindigkeit des ersten Teilgasstroms 14 im Bereich des Bypasses 11 zu erzielen, ist die Gaszuführung 9 im Vergleich zu dem Bypass 11 so ausgestaltet, dass diese einen höheren Strömungswiderstand aufweist. Dadurch wird erreicht, dass eine mittlere Strömungsgeschwindigkeit durch den Bypass 11 eine mittlere Strömungsgeschwindigkeit durch die Messkammer 5 übersteigt.

Die Venturi-Düse 25 wirkt hierbei mit dem höheren Strömungswiderstand zusammen, um eine stabile Durchströmung der Messkammer 5 zu erzeugen.

Die zuvor erläuterten Vorrichtungsmerkmale ermöglichen somit ein Verfahren zur Detektion und/oder Analyse potentiell explosiver Öl-Luft-Gemische in einem überwachten Raum 4, insbesondere in Kurbelräumen oder sonstigen Innenräumen von Verbrennungsmotoren, Kompressoren und sonstigen Kolbenmaschinen, bei dem eine Gasprobe mit Hilfe der Gasfördervorrichtung 27 des Ölnebeldetektors 1 durch eine in dem überwachten Raum 4 / Innenraum angeordnete Ansaugöffnung 8 des Ölnebeldetektors 1 aus dem überwachten Raum 4 / Innenraum entnommen wird. Hierfür muss etwas der Verbrennungsmotor nicht extra aufwändig angepasst werden. Denn die einzige Voraussetzung hierfür ist, dass die Kolbenmaschine, insbesondere der Verbrennungsmotor oder der Kompressor, eine Durchführung bietet, durch die die Gastentnahmevorrichtung 29 des Ölnebeldetektors 1 von außen in den Innenraum geführt und anschließend abgedichtet werden kann.

Wird ein wie zuvor beschriebener Bypass 11 realisiert, kann die Gasprobe in dem Ölnebeldetektor 1 zudem in mindestens zwei Teilgasströme 14, 15 aufgeteilt werden, sodass ein erster Teilgasstrom 14 durch den Bypass 11 unter Umgehung der Messkammer 5 geführt wird, während ein zweiter, insbesondere restlicher, Teilgasstrom 15 durch eine Gaszuführung 9 in die Messkammer 5 geführt wird.

Durch die zuvor beschriebenen Maßnahmen kann dabei erreicht werden, dass kleinere Öltröpfchen mit Durchmessern von weniger als 10 µm, die in der Gasprobe enthalten sind, von dem ersten Teilgasstrom 14 beziehungsweise dem einströmenden Gasstrom 13 separiert werden, und zwar so, dass ein relativer Anteil dieser kleineren Öltröpfchen in dem zweiten Teilgasstrom 15 erhöht wird im Vergleich zu einem relativen Anteil dieser kleineren Öltröpfchen in der Gasprobe. Mit anderen Worten ist somit der relative Anteil von größeren Öltröpfchen in der Messkammer 5 geringer als in der ursprünglichen aus dem Innenraum entnommenen Gasprobe, was vorteilhaft ist für eine präzise Vermessung der besagten kleineren Öltröpfchen, da die größeren Öltröpfchen die Durchlicht- beziehungsweise Streulichtmessungen nicht mehr stören können.

Nochmals zurück kommend auf die eingangs erläuterte optische Messanordnung 44 kann gesagt werden, dass bei der in den Figuren dargestellten Ausgestaltung des Ölnebeldetektors 1 vorgesehen ist: ein erstes Funktionspaar 47 aus einer Lichtquelle 45 und einem Detektor 46 zusammengefasst zu einer ersten entnehmbaren Baueinheit; ein gegenüberliegendes zweites Funktionspaar 48 aus einer Lichtquelle 45 und einem Detektor 46 zusammengefasst zu einer zweiten entnehmbaren Baueinheit; sowie zwei separate, voneinander beabstandet angeordnete Streulichtdetektoren 52 und 71, wobei die beiden Funktionspaare 47, 48 zwei redundante Durchlichtmessungen 6, 72 realisieren und die beiden Streulichtdetektoren 52, 71 jeweils eine Streulichtmessung 73/74 mit Licht realisieren, welches von jeweils einem der Funktionspaare 47/48 ausgesandt und an einer in der Messkammer 5 befindlichen Gasprobe gestreut wurde. Somit können zwei insbesondere zwei redundante Streulichtmessungen gleichzeitig ausgewertet werden und auch die beiden gegenläufigen Durchlichtmessungen 6, 72 sind gleichzeitig auswertbar. Ferner wurde erläutert, dass dabei eine jeweilige Sichtlinie 53 des jeweiligen Streulichtdetektors 52/71 einen Winkel von mindestens 10° mit einer Linie einschließen kann, welche das erste Funktionspaar 47 mit dem zweiten Funktionspaar 48 verbindet. Diese gedachte Linie bildet dabei eine Achse der beiden Durchlichtmessungen 6 und 72.

Zusammenfassend wird zur Verbesserung der Messsicherheit und Messgenauigkeit eines Ölnebeldetektors 1 zur Detektion und/oder Analyse potentiell explosiver Öl-Luftgemische vorgeschlagen, dass der Ölnebeldetektor 1 intern einen Bypass 11 aufweist, mit dem ein erster Teilgasstrom 14, der einen im Vergleich zu einem durch eine Ansaugöffnung 8 von dem Ölnebeldetektor 1 angesaugten Gasstrom 13 erhöhten relativen Anteil an größeren Öltröpfchen von mindestens 10 µm Durchmesser aufweist, an einer optischen Messkammer 5 vorbei geführt wird, sodass ein zweiter, insbesondere restlicher, Teilgasstrom 15, welcher in der Messkammer 5 optisch vermessen wird, vornehmlich kleinere Öltröpfchen von weniger als 10 µm Durchmesser aufweist. Dadurch kann die Sicherheit und Genauigkeit in der Detektion dieser kleineren Öltröpfchen von weniger als 10 µm Durchmesser, die zur Ausbildung potentiell explosiver Gemische führen können, erhöht werden. Ferner wird vorgeschlagen eine Kombination aus einer redundanten Durchlichtmessung 6, 72 und einer mehrfach redundanten Streulichtmessung 73/74, 75/76 zur optischen Detektion der kleineren Öltröpfchen einzusetzen. Durch den Bypass 11 wird die Verschmutzung reduziert. Dies verbessert erheblich die Sicherheit gegen Fehlalarme, verbessert die Messgenauigkeit und verlängert die Reinigungsintervalle.

Wie man in Figur 1 gut erkennt, ist die Gasfördervorrichtung 27 zum aktiven Entnehmen von Gasproben in den Ölnebeldetektor 1 integriert. Die Gasfördervorrichtung 27, die als eine elektrische Gasförderpumpe 28 mit einem elektrischen Antriebsmotor 32 ausgestaltet ist, weist - wie in Figur 3 zu erkennen - eine Lüfterflügelanordnung 31 zum Befördern des Gasstroms 13 auf. Diese Lüfterflügelanordnung 31 ist im Ansaugkanal 7 angeordnet, der seinerseits über die Gasabführung 10 mit der Messkammer 5 verbunden ist. Mit anderen Worten umfasst der Ansaugkanal 7 bei der in den Figuren dargestellten Ausführung die Gaszuführung 9, die Gasabführung 10 als auch den zuvor beschriebenen Bypass 11 und erstreckt sich somit von der Vorkammer 50 ausgehend bis zur Lüfterflügelanordnung 31.

Wie in Figur 15 zu erkennen, ist der elektrische Antriebsmotor 32 der Gasförderpumpe 28 außerhalb des Ansaugkanals 7 angeordnet, um zu verhindern, dass Funken, die am Antriebsmotor 32 entstehen können, zu einer Gasexplosion innerhalb des Ölnebeldetektors 1 führen.

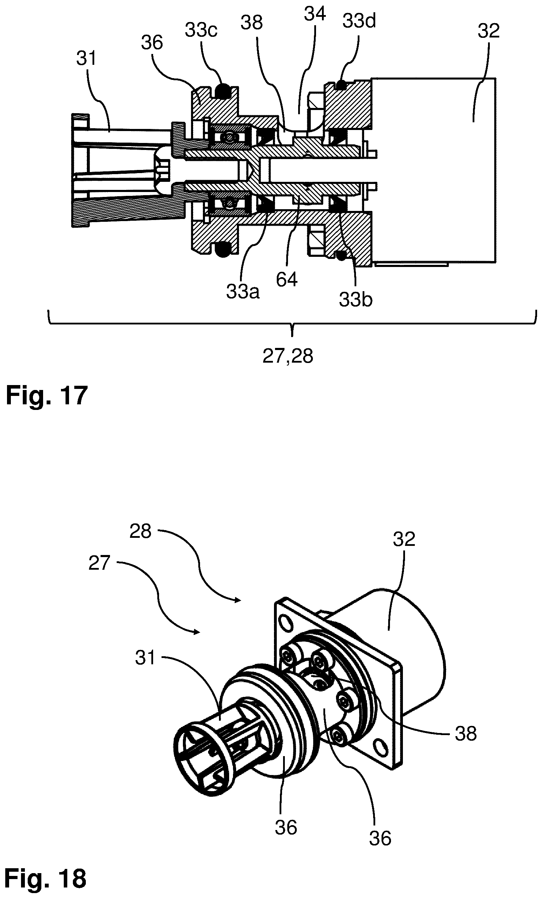

Um hierbei möglichst wenig Gasverlust aus dem Ansaugkanal 7 zu verursachen, ist eine erste Dichtung 33a mit Hilfe eines Elastomerrings zwischen der Lüfterflügelanordnung 31 und dem Antriebsmotor 32 ausgebildet (Vgl. Figur 3). Diese Dichtung 33a dichtet somit den Ansaugkanal 7 gegenüber dem Antriebsmotor 32 ab. Die Dichtung 33a ist dabei als ein Wellendichtring ausgestaltet, der über die Welle 64 geschoben ist, welche das Antriebsmoment des Antriebsmotors 32 auf die Lüfterflügelanordnung 31 überträgt (Vgl. Figur 3 und Figur 17) .

In Gasleckage-Richtung hinter der Dichtung 33a ist ein Zwischenraum 35 gebildet (Vgl. dazu auch Figur 16), das heißt zwischen der Dichtung 33a und dem Antriebsmotor 32. Dieser Zwischenraum 35 wird von einem Patronengehäuse 36 der Gasfördervorrichtung 27 / der Gasförderpumpe 28 ausgebildet. Zudem ist der Zwischenraum 35 mittels einer weiteren Dichtung 33b gegenüber dem Antriebsmotor 32 abgedichtet (Vgl. die Figuren 3 und 17), was verhindert, das Gas, welches die erste Dichtung 33a passiert hat, beispielsweise aufgrund eines Defekts an der Dichtung 33a, bis zum Antriebsmotor 32 vorstoßen kann. Auch die Dichtung 33b ist als ein Wellendichtring ausgestaltet, der über die Welle 64 geschoben ist (Vgl. Figur 3 und Figur 17).

Die gesamte Gasfördervorrichtung 27 / Gasförderpumpe 28 kann mit dem Patronengehäuse 36 in eine hierfür vorgesehene Aufnahme 37 am Ölnebeldetektor 1 eingesetzt werden, wie in Figur 1 illustriert ist. Zudem ist es möglich, das Patronengehäuse 36 und damit den Antriebsmotor 32 ohne Zerlegen des Ölnebeldetektors 1 und ohne Ausbau des Ölnebeldetektors 1 aus dem Kurbelraumgehäuse 3 auszutauschen, sodass eine Wartung der Gasfördervorrichtung 27 besonders einfach und schnell erfolgen kann.

Da nicht ausgeschlossen werden kann, dass im Betrieb eine Leckage von Gas aus dem Ansaugkanal 7 durch die erste Dichtung 33a (oder eine Dichtung 33c) in den Zwischenraum 35 erfolgt, ist dieser mittels einer Entlüftung 34 nach außen entlüftet, wie in den Figuren 17 und 18 zu erkennen ist. Die Entlüftung 34 ist dabei gebildet mittels einer Entlüftungsöffnung 38 des Patronengehäuses 36, welche korrespondierend zu einer Entlüftungsöffnung 60 der Aufnahme 37 ausgebildet ist (Vgl. Figur 16 und 18). Die Entlüftungsöffnung 60 kommuniziert wiederum mit der in den Figuren 9, 11 und 15 zu sehenden Entlüftungsöffnung 62, die zwar von dem Spannelement 63 abgedeckt aber nicht verschlossen wird, sodass ein Entlüftung in die Umgebung realisiert ist, auch bei aufgesetzter Gasentnahmevorrichtung 29. Somit ist der Zwischenraum 35 durch die Entlüftungsöffnungen 38 und 60 hindurch nach außen entlüftet, wenn die Gasfördervorrichtung 27 mit dem Patronengehäuse 36 in die Aufnahme 37 eingesetzt ist.

Das Patronengehäuse 36 bildet somit die Belüftung des Zwischenraums 35 mit aus. Durch die Entlüftung 34 werden somit Gase, welche ausgehend vom Ansaugkanal 7 die erste Dichtung 33a (oder die Dichtung 33c) passiert haben, nach außen in die Aufnahme 37 und von dort in die Umgebung abgeführt, wie anhand der gestrichelten Linie in Figur 16 illustriert ist. Dadurch gelangen diese nicht zum elektrischen Antriebsmotor 32 und die Explosionssicherheit wird somit gewährleistet.

Mit diesen Vorkehrungen lässt sich somit ein sehr sicheres Verfahren zur Detektion explosiver Öl-Luftgemische in einem Innenraum eines Verbrennungsmotors mit Hilfe eines Ölnebeldetektors 1 realisieren. Hierbei wird zunächst eine Gasprobe mit Hilfe der Gasfördervorrichtung 27 aus dem Innenraum entnommen, wobei die Gasprobe mittels einer Lüfterflügelanordnung 31 angesaugt wird, die in dem Ansaugkanal 7 angeordnet ist. Dadurch gelangt die Gasprobe in die Messkammer 5 des Ölnebeldetektors. Gas aus dem Ansaugkanal 7, welches dabei in Richtung des Antriebsmotors 32 der Gasfördervorrichtung 27, insbesondere durch die erste Dichtung 33a (oder die Dichtung 33c) hindurch, aus dem Ansaugkanal 7 entweicht, wird dabei mittels der zuvor beschriebenen Entlüftung 34 nach außen abgeführt. Dadurch kann sich kein explosives Gemisch in unmittelbarer Nähe des elektrischen Antriebsmotors 32 mehr ausbilden, insbesondere aufgrund der zweiten Dichtung 33b (oder eine Dichtung 33d).

Zusammenfassend wird zur Verbesserung der Handhabung wie der Betriebssicherheit eines optischen Ölnebeldetektors 1 zur optischen Detektion von feinem Ölnebel 2 vorgeschlagen, dass eine optisch zu untersuchende Gasprobe von dem Ölnebeldetektor 1 mittels einer elektrischen Gasförderpumpe 28 in eine Messkammer 5 gefördert wird und dass dabei ein elektrischer Antriebsmotor 32 der Gasförderpumpe 28 mittels eines nach außen entlüfteten Zwischenraums 35 von einem Ansaugkanal 7 des Ölnebeldetektors 1 entkoppelt ist, durch den die Gasprobe mittels der Gasförderpumpe 28 gefördert wird.

Der in den Figuren illustrierte Ölnebeldetektor 1 ist nicht nur zum Einsetzen in ein Gehäuse 3, welches einen Innenraum 4 begrenzt, eingerichtet, wie in Figur 4 illustriert, sondern ferner dazu, eine Testgaszuführung 41 anzusteuern, um so ein Testgas von außen in den Innenraum 4 einzuführen. Wie im Folgenden erläutert wird, dient diese Funktionalität dazu, eine Testmessung zu ermöglichen, mit Hilfe derer die korrekte Funktionsweise des Ölnebeldetektors 1 sicher überprüft werden kann.

Bei dem in den Figuren gezeigten Beispiel bildet der Ölnebeldetektor 1, genauer dessen Gasentnahmevorrichtung 29, die Testgaszuführung 41 selbst aus, wie anhand der Figur 8 gut zu erkennen ist:
Die Gasentnahmevorrichtung 29 bietet einen ersten Kanal 42, der als Einlasskanal 51 für ein Testgas dient. Indem der Ölnebeldetektor 1 das in den Figuren 5, 6 und 8 erkennbare Einlassventil 56 für das Testgas ansteuert, kann der Ölnebeldetektor 1 somit einen (mit Pfeilen in Figur 8 illustrierten) Testgasstrom 59 durch das Einlassventil 56 und den Einlasskanal 51 bis in den Innenraum 4 bewirken. Hierbei endet der Einlasskanal 51 gerade im Bereich der Einlassöffnung 8 der Gasentnahmevorrichtung 29. Dies bewirkt, dass das Testgas durch die Einlassöffnung 8 und einen sich anschließenden zweiten Kanal 43, als Teil des zuvor erläuterten Ansaugkanals 7, strömen kann und damit denselben Weg bis zur Messkammer 5 zurück legt, den eine Gasprobe im normalen Messbetrieb von dem Kurbelinnenraum 4 bis zur Messkammer 5 zurücklegt.

Während der erste Kanal 42 somit dem Zuführen eines definierten Testgasstroms 5 in den Innenraum 4 dient, ist der Zweck des zweiten Kanals 43 das Abführen der Testgasprobe aus dem Innenraum 4 in die außen liegende Messkammer 5. Hierbei verbindet der zweite Kanal 43 die Ansaugöffnung 8 mit der Messkammer 5 bildet somit den zuvor erläuterten Ansaugkanal 7 mit aus. In Figur 8 ist zudem zu erkenne, dass der erste Kanal 42 und der zweite Kanal 43 mittels einer Trennwand 61 separiert sind, sodass Testgas insbesondere in einem kontinuierlichen Betrieb mit einer vorgegebenen Volumenrate durch die Testgaszuführung 41 in den Innenraum 4 eingespült werden kann.

Denn wie bereits erläutert wurde, weist der Ölnebeldetektor 1 zudem eine Gasfördervorrichtung 27 in Form einer elektrischen Gasförderpumpe 28 zum aktiven Entnehmen von Gasproben aus dem Innenraum 4 auf sowie die besagte Gasentnahmevorrichtung 29.

Mit der in den Figuren 4 bis 8 illustrierten Gasentnahmevorrichtung 29, die zur Entnahme von Gasproben aus dem Innenraum 4 und durch das Gehäuse 3 hindurch eingerichtet ist, kann somit das durch die Testgaszuführung 41 in den Innenraum 4 einströmende Testgas wieder entnommen und in die Messkammer 5 überführt werden, um dort eine optische Testmessung an dem Testgas durchzuführen. Dabei wird das Testgas über die Gasförderpumpe 28 angesaugt und zwar genau gleich wie eine Gasprobe, die mit Hilfe der Gasentnahmevorrichtung 29 im Normalbetrieb aus dem Innenraum 4 entnommen wird.

Wie man gut in der Längsschnittansicht der Figur 8 erkennt, ist die mittels des Einlasskanals 51 und dem Einlassventil 56 ausgestaltete Testgaszuführung 41 so ausgestaltet, dass in einem Testmessbetrieb das Testgas den gleichen Weg vom Innenraum 4 bis zur Messkammer 5 durch die Gasentnahmevorrichtung 29 hindurch zurücklegt wie eine Gasprobe, die im normalen Messbetrieb von dem Ölnebeldetektor 1 aus dem Innenraum 4 entnommen und optisch vermessen wird.

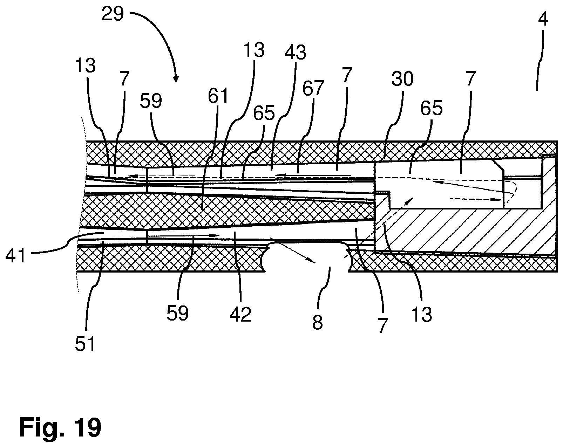

Die Figur 19 zeigt eine weitere mögliche Ausgestaltung der Gasentnahmevorrichtung 29, wobei im Vergleich zu Figur 8 in Figur 19 der Testgasstrom 59 von links nach rechts in den Innenraum 4 geleitet wird. Im Unterschied zur Ausgestaltung gemäß Figur 8 ist in Figur 19 vorgesehen, dass der erste Kanal 42 der Gasentnahmevorrichtung 29 derart mit der Ansaugöffnung 8 in Verbindung steht, dass der Testgasstrom 59 nach Passieren der Ansaugöffnung 8 exakt denjenigen Weg bis in die Messkammer 5 nimmt, den der eigentlich zu vermessende Gasstrom 13 im normalen Betrieb des Ölnebeldetektors 1 zurücklegt. Dadurch wird also sichergestellt, dass das Zuführen des Testgases und die damit durchgeführte Testgasmessung überprüft, ob der Weg von der Ansaugöffnung 8 bis zur Messkammer 5 vollständig frei ist.

Die Ausgestaltung der Figur 19 ist somit dadurch gekennzeichnet, dass die Testgaszuführung 41, genauer deren erster Kanal 42, von der Ansaugöffnung 8 mit ausgebildet wird. Dadurch wird erreicht, dass der Testgasstrom 59 nach Passieren der Ansaugöffnung 8 denselben Weg zurücklegt in die Messkammer 5 wie eine Gasprobe, welche im Normalbetrieb, aus dem überwachten Innenraum 4 mit Hilfe der Gasentnahmevorrichtung 29 entnommen wird. Ist beispielsweise die Ansaugöffnung 8 blockiert, so kann dies mit einer Testgasmessung detektiert werden. Bei einer Ausgestaltung wie in Figur 8 besteht hingegen die Gefahr, dass der Testgasstrom 59 ausgehend vom ersten Kanal 42 auch dann noch in Messkammer 5 gelangen kann, wenn die Ansaugöffnung 8 blockiert ist. Mit anderen Worten ist somit mit einer Ausgestaltung wie in Figur 19 illustriert eine höhere Sicherheitsstufe hinsichtlich der Überprüfung der Funktionstüchtigkeit des Ölnebeldetektors 1 erreichbar, im Vergleich zur Ausgestaltung gemäß Figur 8, bei welcher zwar eine Verstopfung innerhalb des zweiten Kanals 43 (etwa durch eine flüssige Ölansammlung) detektierbar ist aber nicht zwingend auch eine Blockade der Ansaugöffnung 8.

Wie bereits zuvor erläutert wurde, ist die Messkammer 5 mit dem Ansaugkanal 7 verbunden, der - wie die Figuren 5, 6 und 8 illustrieren - in der Ansaugöffnung 8 mündet, die sich in der Einbausituation innerhalb des Innenraums 4 befindet, während die Messkammer 5 außerhalb des Innenraums 4 angeordnet ist. Die Testgaszuführung 41 mündet nun gerade im Bereich der Ansaugöffnung 8, wie man in Figur 8 gut erkennt. An der Mündungsstelle vereinigt sich somit der Testgasstrom mit dem zuvor erläuterten Gasstrom 13 (bzw. ersetzt diesen im Testmessbetrieb), der aus der Umgebung der Ansaugöffnung 8 herrührt. Diese Ausgestaltung hat den Vorteil, dass über die Testgaszuführung 41, d.h. insbesondere den Einlasskanal 51, in den Innenraum 4 zugeführtes Testgas durch die Ansaugöffnung 8 aus dem Innenraum 4 wieder mit dem Ölnebeldetektor 1 entnehmbar ist. Dadurch kann also gezielt überprüft werden, ob die Gasentnahmevorrichtung 29 auch tatsächlich eine Gasprobe aus dem Innenraum 4 zur Messkammer 5 weiterleiten kann. Anders als bei einer nur externen Zuführung von Testgas in die Messkammer 5, kann also in dem besagten Testbetrieb nicht nur die korrekte Funktionsweise der Messkammer, sondern auch die korrekte Funktionsweise des Transports von Gasproben aus dem Innenraum durch die Gasentnahmevorrichtung 29 bis in die Messkammer 5 überprüft werden. Dies erhöht damit die Sicherheit der Überprüfung.

Dadurch, dass die Testgaszuführung 41 in die Gasentnahmevorrichtung 29 integriert ist, muss somit nur eine Durchführung 40 in dem Gehäuse 3 vorgesehen werden. Hierbei ist die Gasentnahmevorrichtung 29 gerade so ausgestaltet, dass diese durch die Öffnung 40 mit einem ¾ Zoll-Gewinde passt. Somit kann die Messanordnung einfach bei einer bestehenden Maschine nachgerüstet werden, da derartige Zugangsöffnungen üblich sind.

Der Ölnebeldetektor 1 kann darüber hinaus selbsttätig einen Selbsttest seiner Funktionsweise auf Basis einer an einer Gasprobe des Testgases in der Messkammer 5 vorgenommenen optischen Messung ausführen. Hierbei gibt der Ölnebeldetektor 1 auch das Ergebnis des Selbsttests in Form eines Benutzerhinweises aus und speichert dieses in einem internen Speicher ab.

Mit der in den Figuren gezeigten Ausführung des Ölnebeldetektors 1 lässt sich somit ein Verfahren zur Sicherheitsüberprüfung des Ölnebeldetektors 1 umsetzen, bei dem der Ölnebeldetektor 1 durch Ansteuern des Einlassventils 56 der Testgaszuführung 41 das Testgas in den von dem Ölnebeldetektor 1 überwachten Innenraum 4 einführt. Anschließend entnimmt der Ölnebeldetektor 1 mit Hilfe seiner Gasfördervorrichtung 27 Testgas wieder aus dem Innenraum 4 und vermisst diese anschließend optisch in der Messkammer 5 im Rahmen der zuvor diskutierten Testmessung. Dabei überprüft der Ölnebeldetektor 1 seine eigene korrekte Funktionsweise anhand eines Messergebnisses dieser Testmessung. Beispielsweise kann der Ölnebeldetektor 1 überprüfen, ob - für eine von ihm eingestellte Volumenrate des Testgasstroms 59 eine Mindestmenge / Mindestkonzentration an Testgas in der Messkammer detektiert werden kann. Ist dies der Fall, kann geschlossen werden, dass die Gasentnahmevorrichtung 29 einen für einen sicheren Betrieb notwendigen Mindestvolumenstrom an Testgas passieren lässt.

Bei diesem Verfahren fördert die Gasfördervorrichtung 27 somit durch die Gasentnahmevorrichtung 29 Testgas in die Messkammer 5 entlang desselben Weges, entlang welchem im Normalbetrieb Gasproben aus der Atmosphäre des Innenraums 4 in die Messkammer 5 gelangen.

Zur Verbesserung der Sicherheit der optischen Detektion von feinem Ölnebel 2 mit Hilfe eines Ölnebeldetektors 1 wird somit vorgeschlagen, dass ein Testgas mittels einer von dem Ölnebeldetektor 1 bereit gestellten oder zumindest angesteuerten Testgaszuführung 41 in einen Innenraum 4 eingespült wird und anschließend eine Gasprobe des Testgases durch eine Gasentnahmevorrichtung 29 des Ölnebeldetektors 1 hindurch wieder aus dem Innenraum 4 entnommen wird, um diese mit Hilfe des Ölnebeldetektors 1 optisch zu vermessen. Hierbei folgt das Testgas bevorzugt einem Strömungsweg bis zu einer optischen Messkammer 5 des Ölnebeldetektors 1, den eine Gasprobe aus dem Innenraum 4 in einem Normalbetrieb des Ölnebeldetektors 1 ausgehend von einer Ansaugöffnung 8 nimmt.

### Bezugszeichenliste

- 1: Ölnebeldetektor
- 2: Ölnebel
- 3: Gehäuse (insbesondere Kurbelraumgehäuse)
- 4: überwachter Raum, insbesondere Innenraum (z.B. Kurbelraum)
- 5: Messkammer
- 6: optische Messstrecke
- 7: Ansaugkanal
- 8: Ansaugöffnung
- 9: Gaszuführung
- 10: Gasabführung
- 11: Bypass
- 12: Verzweigungsstelle
- 13: Gasstrom (durch 7)
- 14: erster Teilgasstrom (von 13, wird an 5 vorbei geführt)
- 15: zweiter Teilgasstrom (von 13, gelangt in 5)
- 16a: Strömungsrichtung (von 13)
- 16b: Strömungsrichtung (von 13)
- 16c: Strömungsrichtung (von 13)
- 16d: Strömungsrichtung (von 13)
- 16e: Strömungsrichtung (von 13)
- 17: Beschleunigungsabschnitt (von 7)
- 18: Beschleunigungsrichtung (von 17)
- 19: Anstieg
- 20: Prallfläche
- 21: Umlenkfläche
- 22: Fangtrichter
- 23: Richtungsumkehr
- 24: Vereinigungsstelle
- 25: Venturi-Düse
- 26: Querschnittsverengung
- 27: Gasfördervorrichtung
- 28: Gasförderpumpe
- 29: Gasentnahmevorrichtung
- 30: Ansaugrohr
- 31: Lüfterflügelanordnung
- 32: Antriebsmotor (von 27)
- 33a: (erste) Dichtung
- 33b: (zweite) Dichtung
- 33c: Dichtung
- 33d: Dichtung
- 34: Entlüftung
- 35: Zwischenraum (von außen belüftet)
- 36: Patronengehäuse (von 32)
- 37: Aufnahme (für 36)
- 38: Entlüftungsöffnung (von 36)
- 39: Gehäuse
- 40: Durchführung (in 3 für 29)
- 41: Testgaszuführung
- 42: erster Kanal (von 29)
- 43: zweiter Kanal (von 29)5
- 44: optische Messanordnung
- 45: Lichtquelle
- 46: Lichtdetektor
- 47: erstes Funktionspaar (aus 45 und 46)
- 48: zweites Funktionspaar (aus 45 und 46)
- 49: Aufnahme (für 47/48/52/71)
- 50: Vorkammer
- 51: Einlasskanal (für Testgas)
- 52: weiterer Lichtdetektor (zur Streulichtmessung)
- 53: Sichtlinie (von 52/71)
- 54a: (erste) Austrittsöffnung
- 54b: (zweite) Austrittsöffnung
- 55: Austrittskanal
- 56: Einlassventil (für Testgas)
- 57: Ausflussöffnung
- 58: Schnittstelle (für 29)
- 59: Testgasstrom
- 60: Entlüftungsöffnung (von 37)
- 61: Trennwand
- 62: Entlüftungsöffnung
- 63: Spannelement
- 64: Welle
- 65: Rückführstrom
- 66: Rückführöffnung
- 67: Rückführkanal
- 68: Wandung von 37
- 69: Ansaugöffnung von 70
- 70: Messteil
- 71: weiterer Lichtdetektor (zur Streulichtmessung)
- 72: optische Messstrecke
- 73: optische Messstrecke
- 74: optische Messstrecke
- 75: optische Messstrecke
- 76: optische Messstrecke

## Patentansprüche

1. **Ölnebeldetektor** (1) zur Detektion und/oder Analyse von Öl-Luft-Gemischen, insbesondere zum Einsetzen in ein Kurbelraumgehäuse (3), welches einen Kurbelraum (4) einer Kolbenmaschine begrenzt, mit
- einer Messkammer (5), die mindestens eine optische Messstrecke (6, 72) zur optischen Detektion eines Ölnebels (2) ausbildet, wobei die Messkammer (5) eine optische Messanordnung (44) von Lichtquellen (45), beispielsweise LEDs, und Lichtdetektoren (46), beispielsweise Photodioden, aufweist,
- die optische Messanordnung (44) ein erstes Funktionspaar (47) und ein zweites Funktionspaar (48) aus je einer Lichtquelle (45) und einem Lichtdetektor (46) umfasst,
- die Lichtquelle (45) des ersten Funktionspaars (47) mit dem Lichtdetektor (46) des zweiten Funktionspaars (48) eine erste optische Messtrecke (6) ausbildet, während die Lichtquelle (45) des zweiten Funktionspaars (48) mit dem Lichtdetektor (46) des ersten Funktionspaars (47) eine zweite optische Messtrecke (72) ausbildet, und
- die optische Messanordnung (44) zur gleichzeitigen Durchführung einer Durchlichtmessung und einer Streulichtmessung, jeweils an einer in der Messkammer (5) befindlichen Gasprobe, eingerichtet ist;
**dadurch gekennzeichnet dass** zur Ermöglichung einer redundanten Durchlichtmessung die erste und die zweite optische Messstrecke (6, 72) in zueinander entgegengesetzten Richtungen verlaufen und dieselbe Messstrecke durch die Messkammer (5) zurücklegen.

2. Ölnebeldetektor (1) nach Anspruch 1, wobei die beiden Funktionspaare (47, 48) jeweils baulich als eine Einheit ausgestaltet sind, die jeweils in eine Aufnahme (49) der Messkammer (5), vorzugsweise herausnehmbar, eingesetzt sind,
- vorzugsweise wobei sich die beiden Aufnahmen (49) derart gegenüberliegen, dass die erste und zweite optische Messstrecke (6, 72) durch die Messkammer (5) verlaufen.

3. Ölnebeldetektor (1) nach Anspruch 1 oder 2, wobei die optische Messanordnung (44) mindestens einen weiteren Lichtdetektor (52) zur Streulichtmessung aufweist, der eine Sichtlinie (53) ausbildet, welche einen Winkel von mindestens 10° zur ersten optischen Messstrecke (6) und/oder zur zweiten optischen Messstrecke (72) einschließt.

4. Ölnebeldetektor (1) nach Anspruch 3, wobei mindestens ein zweiter weiterer Lichtdetektor (71) zur Durchführung mindestens einer zweiten Streulichtmessung vorgesehen ist, die redundant ist/sind zu einer mittels des weiteren Lichtdetektors (52) durchgeführten ersten Streulichtmessung.

5. Ölnebeldetektor (1) nach einem der vorhergehenden Ansprüche, wobei der Ölnebeldetektor (1) eine Berechnungseinheit aufweist, die dazu eingerichtet ist, zwei unabhängige Durchlichtmessungen mittels der ersten und zweiten optischen Messstrecke (6, 72), insbesondere gleichzeitig, auszuwerten.

6. Ölnebeldetektor (1) nach einem der vorhergehenden Ansprüche, wobei der Ölnebeldetektor (1) eine, insbesondere die, Berechnungseinheit, insbesondere in Form einer Elektronik-Einheit, aufweist, die dazu eingerichtet ist, eine Lichtabsorption auf Basis der Durchlichtmessung sowie eine Streulichtintensität auf Basis der Streulichtmessung quantitativ zu bestimmen.

7. Ölnebeldetektor (1) nach Anspruch 6, wobei die Berechnungseinheit dazu eingerichtet ist, anhand der bestimmten Lichtabsorption und der bestimmten Streulichtintensität Messsignale, die einem Wasseranteil einer Gasprobe in der Messkammer (5) zuordenbar sind, von Messsignalen zu unterscheiden, die einem Anteil eines Aerosols von Öltröpfchen, insbesondere von weniger als 10 µm Durchmesser, der Gasprobe zuordenbar sind,
- insbesondere wobei die Berechnungseinheit dazu eingerichtet ist, die bestimmte Streulichtintensität zur Differenzierung der beiden Messsignale zu verwenden und/oder
- auf Basis der bestimmten Lichtabsorption und/oder der bestimmten Streulichtintensität auf die Anwesenheit des Aerosols von Öltröpfchen zu schließen und ein entsprechendes Warnsignal auszugeben.

8. Ölnebeldetektor (1) nach einem der vorhergehenden Ansprüche, wobei die Berechnungseinheit dazu eingerichtet ist,
- anhand von mit der zweiten optischen Messstrecke (72) gewonnenen Messdaten eine Plausibilitätsprüfung durchzuführen in Bezug auf Messdaten, die mit der ersten optischen Messstrecke (6) gewonnen wurden und/oder
- anhand von mit der zweiten Streulichtmessung/dem zweiten Streulichtdetektor (71) gewonnenen Messdaten eine Plausibilitätsprüfung durchzuführen in Bezug auf Messdaten, die mit der ersten Streulichtmessung/dem ersten Streulichtdetektor (52) gewonnen wurden.

9. **Kolbenmaschine** mit einem einen Kurbelraum (4) begrenzenden Kurbelraumgehäuse (3) und einem Ölnebeldetektor (1) gemäß einem der Ansprüche 1 bis 8, wobei der Ölnebeldetektor (1) zur Entnahme von Gasproben aus dem Kurbelraum (4) eingerichtet ist,
- vorzugsweise wobei hierzu eine Gasentnahmevorrichtung (29) des Ölnebeldetektors (1), beispielsweise ein Ansaugrohr (30), durch das Kurbelraumgehäuse (3) in den Kurbelraum (4) geführt ist, wobei der Ölnebeldetektor (1), insbesondere die Messkammer (5) und/oder eine Gasfördervorrichtung (27) des Ölnebeldetektors (1), von außen zugänglich ist.

10. **Verfahren zum Betrieb eines Ölnebeldetektors** (1), insbesondere ausgestaltet gemäß einem der Ansprüche 1 bis 9, mit einer Messkammer (5), die mindestens eine optische Messstrecke (6, 72) zur optischen Detektion eines Ölnebels (2) ausbildet, **dadurch gekennzeichnet,**
- **dass** zur Detektion des Ölnebels (2) eine erste und eine zweite optische Messstrecke (6, 72), die gegenläufig durch die Messkammer (5) verlaufen und dieselbe Messstrecke durch die Messkammer (5) zurücklegen, gleichzeitig oder alternierend für eine redundante Durchlichtmessung ausgewertet werden,
- **dass** zusätzlich mindestens eine Streulichtmessung unter Verwendung von Licht der ersten oder zweiten optischen Messstrecke (6, 72) zur Detektion des Ölnebels (2) eingesetzt wird,
- wobei die Durchlichtmessung und die Streulichtmessung gleichzeitig durchgeführt werden.

11. Verfahren gemäß Anspruch 10 wobei zur Detektion des Ölnebels (2) mindestens eine dritte optische Messstrecke (73) und eine vierte optische Messstrecke (74), die jeweils in einem Winkel, vorzugsweise von wenigstens 10°, zu der ersten oder zweiten Messstrecke (6, 72) verlaufen, gleichzeitig oder alternierend für eine mehrfach redundante Streulichtmessung ausgewertet werden.

## Claims

1. **Oil-mist detector** (1) for detecting and/or analysing oil-air mixtures, in particular for use in a crank chamber housing (3) that delimits a crank chamber (4) of a piston machine, having
- a measuring chamber (5) that forms at least one optical measuring path (6, 72) for the optical detection of an oil mist (2), wherein the measuring chamber (5) contains an optical measuring arrangement (44) of light sources (45), for example LEDs, and light detectors (46), for example photodiodes,
- the optical measuring arrangement (44) comprises a first functional pair (47) and a second functional pair (48) consisting of in each case a light source (45) and a light detector (46),
- the light source (45) of the first functional pair (47) forms a first optical measuring path (6) with the light detector (46) of the second functional pair (48), while the light source (45) of the second functional pair (48) forms a second optical measuring path (72) with the light detector (46) of the first functional pair (47), and
- the optical measuring arrangement (44) is configured to simultaneously carry out a transmitted light measurement and a scattered light measurement in each case on a gas sample located in the measuring chamber (5) ;
**characterized in that**, in order to enable a redundant transmitted light measurement, the first and the second optical measuring path (6, 72) run in mutually opposing directions and cover the same measuring path through the measuring chamber (5).

2. Oil-mist detector (1) according to Claim 1, wherein the two functional pairs (47, 48) are each designed structurally as a unit that are each able to be inserted, preferably removably, into a receptacle (49) in the measuring chamber (5),
- preferably wherein the two receptacles (49) lie opposite one another such that the first and second optical measuring path (6, 72) run through the measuring chamber (5).

3. Oil-mist detector (1) according to Claim 1 or 2, wherein the optical measuring arrangement (44) has at least one further light detector (52) for scattered light measurement that forms a line of sight (53) that encloses an angle of at least 10° with respect to the first optical measuring path (6) and/or with respect to the second optical measuring path (72).

4. Oil-mist detector (1) according to Claim 3, wherein provision is made for at least one second further light detector (71) for carrying out at least one second scattered light measurement that is/are redundant to a first scattered light measurement carried out by way of the further light detector (52).

5. Oil-mist detector (1) according to one of the preceding claims, wherein the oil-mist detector (1) has a computing unit that is configured to evaluate, in particular simultaneously, two independent transmitted light measurements by way of the first and second optical measuring path (6, 72).

6. Oil-mist detector (1) according to one of the preceding claims, wherein the oil-mist detector (1) has an, in particular the, computing unit, in particular in the form of an electronic unit, which is configured to determine quantitatively a light absorption on the basis of the transmitted light measurement and a scattered light intensity on the basis of the scattered light measurement.

7. Oil-mist detector (1) according to Claim 6, wherein the computing unit is configured, based on the determined light absorption and the determined scattered light intensity, to distinguish measured signals that are able to be assigned to a water component of a gas sample in the measuring chamber (5) from measured signals that are able to be assigned to a component of an aerosol of oil droplets, in particular of less than 10 µm in diameter, in the gas sample,
- in particular wherein the computing unit is configured to use the determined scattered light intensity to differentiate between the two measured signals, and/or
- to infer the presence of the aerosol of oil droplets on the basis of the determined light absorption and/or the determined scattered light intensity and to output a corresponding warning signal.

8. Oil-mist detector (1) according to one of the preceding claims, wherein the computing unit is configured,
- based on measured data obtained using the second optical measuring path (72), to carry out a plausibility check in relation to measured data that have been obtained using the first optical measuring path (6)
and/or
- based on measured data obtained using the second scattered light measurement/the second scattered light detector (71), to carry out a plausibility check in relation to measured data that have been obtained using the first scattered light measurement/the first scattered light detector (52).

9. **Piston machine** having a crank chamber housing (3) delimiting a crank chamber (4) and having an oil-mist detector (1) according to one of Claims 1 to 8, wherein the oil-mist detector (1) is configured to extract gas samples from the crank chamber (4),
- preferably wherein, for this purpose, a gas extraction device (29) of the oil-mist detector (1), for example an intake pipe (30), is guided through the crank chamber housing (3) into the crank chamber (4), wherein the oil-mist detector (1), in particular the measuring chamber (5) and/or a gas conveyor device (27) of the oil-mist detector (1), is externally accessible.

10. **Method for operating an oil-mist detector** (1), in particular designed according to one of Claims 1 to 9, having a measuring chamber (5) that forms at least one optical measuring path (6, 72) for the optical detection of an oil mist (2), **characterized**
- **in that**, in order to detect the oil mist (2), a first and a second optical measuring path (6, 72) that run through the measuring chamber (5) in opposing directions and cover the same measuring path through the measuring chamber (5) are evaluated simultaneously or alternately for a redundant transmitted light measurement,
- **in that** use is additionally made of at least one scattered light measurement using light from the first or second optical measuring path (6, 72) to detect the oil mist (2),
- wherein the transmitted light measurement and the scattered light measurement are carried out simultaneously.

11. Method according to Claim 10, wherein, in order to detect the oil mist (2), at least a third optical measuring path (73) and a fourth optical measuring path (74) that each run at an angle, preferably of at least 10°, with respect to the first or second measuring path (6, 72) are evaluated simultaneously or alternately for a multiple-redundant scattered light measurement.

## Revendications

1. **Détecteur de brouillard d'huile** (1) pour la détection et ou l'analyse de mélanges huile/air, en particulier à insérer dans un carter de chambre de piston (3) qui délimite une chambre de piston d'une machine à piston, comprenant
- une chambre de mesure (5) qui forme au moins une section de mesure optique (6, 72) pour la détection optique d'un brouillard d'huile (2),
- cette chambre de mesure (5) comportant un dispositif de mesure optique (44) de sources lumineuses (45), par exemple de LED, et de détecteurs de lumière (46), par exemple de photodiodes,
- ce dispositif de mesure optique (44) comprenant une première paire fonctionnelle (47) et une deuxième paire fonctionnelle (48) constituées chacune d'une source lumineuse (45) et d'un détecteur de lumière (46),
- la source lumineuse (45) de la première paire fonctionnelle (47) formant avec le détecteur de lumière (46) de la deuxième paire fonctionnelle (48) une première section de mesure optique (6), tandis que la source lumineuse (45) de la deuxième paire fonctionnelle (48) forme avec le détecteur de lumière (46) de la première paire fonctionnelle (47) une deuxième section de mesure optique (72), et
- le dispositif de mesure optique (44) étant agencé pour réaliser simultanément une mesure de lumière transmise et une mesure de lumière dispersée sur un échantillon de gaz se trouvant dans la chambre de mesure (5),
**caractérisé en ce que**, pour permettre une mesure de lumière transmise redondante, la première et la deuxième section de mesure optique (6, 72) sont orientées dans des directions opposées entre elles et parcourent la même section de mesure à travers la chambre de mesure (5).

2. Détecteur de brouillard d'huile (1) selon la revendication 1, dans lequel les deux paires fonctionnelles (47, 48) sont configurées chacune structurellement comme une unité et sont insérées chacune, de préférence de manière amovible, dans un logement (49) de la chambre de mesure (5).
- de préférence les deux logements (49) se faisant face de telle sorte que les première et deuxième sections de mesure optiques (6, 72) courent à travers la chambre de mesure (5).

3. Détecteur de brouillard d'huile (1) selon la revendication 1 ou 2, dans lequel le dispositif de mesure optique (44) comporte, pour mesurer la lumière dispersée, au moins un autre détecteur de lumière (52) qui constitue une ligne de visée (53) qui forme un angle d'au moins 10° par rapport à la première section de mesure optique (6) et/ou à la deuxième section de mesure optique (72).

4. Détecteur de brouillard d'huile (1) selon la revendication 3, dans lequel il est prévu au moins un deuxième détecteur de lumière (71) pour réaliser au moins une deuxième mesure de lumière dispersée qui est redondante par rapport à une première mesure de lumière dispersée réalisée au moyen de l'autre détecteur de lumière (52).

5. Détecteur de brouillard d'huile (1) selon une des revendications précédentes, lequel détecteur de brouillard d'huile (1) comporte une unité de calcul qui est conçue pour évaluer, en particulier simultanément, deux mesures de lumière transmise indépendantes au moyen des première et deuxième sections de mesure optiques (6, 72).

6. Détecteur de brouillard d'huile (1) selon une des revendications précédentes, lequel détecteur de brouillard d'huile (1) comporte une unité de calcul, en particulier l'unité de calcul, en particulier sous la forme d'une unité électronique, qui est agencée pour déterminer quantitativement une absorption de lumière sur la base de la mesure de lumière transmise ainsi qu'une intensité de lumière dispersée sur la base de la mesure de lumière dispersée.

7. Détecteur de brouillard d'huile (1) selon la revendication 6, dans lequel l'unité de calcul est conçue pour distinguer, à l'aide de l'absorption de lumière déterminée et de l'intensité de lumière dispersée déterminée, les signaux de mesure qui peuvent être attribués à une teneur en eau d'un échantillon de gaz dans la chambre de mesure (5) des signaux de mesure qui peuvent être attribués à une teneur en un aérosol de gouttelettes d'huile, en particulier de moins de 10 µm de diamètre, de l'échantillon de gaz,
- en particulier dans lequel l'unité de calcul est agencée pour utiliser l'intensité de lumière dispersée déterminée pour différencier les deux signaux de mesure et/ou
- pour conclure à la présence de l'aérosol de gouttelettes d'huile sur la base de l'absorption de lumière déterminée et/ou de l'intensité de lumière dispersée déterminée et pour émettre un signal d'alarme correspondant.

8. Détecteur de brouillard d'huile (1) selon une des revendications précédentes, dans lequel l'unité de calcul est agencée
- pour réaliser, à l'aide de données de mesure acquises avec la deuxième section de mesure optique (72), un contrôle de vraisemblance par rapport à des données de mesure qui ont été acquises avec la première section de mesure optique (6) et/ou
- pour réaliser, à l'aide de données de mesure acquises avec la deuxième mesure de lumière dispersée/le deuxième détecteur de lumière dispersée (71), un contrôle de vraisemblance par rapport à des données de mesure qui ont été acquises avec la première mesure de lumière dispersée/le premier détecteur de lumière dispersée (52).

9. **Machine à piston** avec un carter de chambre de piston (3) délimitant une chambre de piston (4) et un détecteur de brouillard d'huile (1) selon une des revendications 1 à 8, dans laquelle le détecteur de brouillard d'huile (1) est conçu pour prélever des échantillons de gaz dans la chambre de piston (4),
- un dispositif de prélèvement de gaz (29) du détecteur de brouillard d'huile (1), par exemple un tube d'aspiration (30), étant de préférence conduit pour cela dans la chambre de piston (4) à travers le carter de chambre de piston (3), le détecteur de brouillard d'huile (1), en particulier la chambre de mesure (5) et/ou un dispositif de transport de gaz (27) du détecteur de brouillard d'huile (1), étant accessibles de l'extérieur.

10. **Procédé d'utilisation d'un détecteur de brouillard d'huile** (1), en particulier configuré selon une des revendications 1 à 9, avec une chambre de mesure (5) qui forme au moins une section de mesure (6, 72) pour la détection optique d'un brouillard d'huile (2), **caractérisé en ce que,**
- pour la détection du brouillard d'huile (2), une première et une deuxième section de mesure optique (6, 72) qui courent en sens contraires à travers la chambre de mesure (5) et parcourent la même section de mesure à travers la chambre de mesure (5) sont évaluées simultanément ou alternativement pour une mesure de lumière transmise redondante et que,
- de plus, au moins une mesure de lumière dispersée est effectuée en utilisant la lumière des première ou deuxième sections de mesure optiques (6, 72) pour la détection du brouillard d'huile (2),
- la mesure de lumière transmise et la mesure de lumière dispersée étant réalisées simultanément.

11. Procédé selon la revendication 10, selon lequel, pour la détection du brouillard d'huile (2), au moins une troisième section de mesure optique (73) et une quatrième section de mesure optique (74) qui courent chacune sous un angle de préférence d'au moins 10° par rapport à la première ou à la deuxième section de mesure (6, 72) sont évaluées simultanément ou alternativement pour une mesure de lumière dispersée à redondance multiple.
